# EUROPEAN PATENT APPLICATION

(11) **EP 3 805 235 A1**
(43) Date of publication of application: **14.04.2021**
(21) Application number: 19810341.8
(22) Date of filing: 30.05.2019
(51) Int. Cl.: C07D 519/00, A61K 47/54, A61K 47/66, A61K 49/04, A61K 51/00, A61K 51/04, A61P 35/00

(54) **HALOGENATED BIOTIN-MODIFIED DIMER AND USE THEREOF**

(30) Priority: 30.05.2018 JP 2018103808
(71) Applicant: The University of Tokyo, Bunkyo-ku, Tokyo 113-8654 (JP); Savid Therapeutics Inc., Tokyo 104-0054 (JP)
(72) Inventor: KANAI Motomu, Tokyo 113-8654 (JP); SHIMIZU Yohei, Tokyo 113-8654 (JP); YAMATSUGU Kenzo, Tokyo 113-8654 (JP); TATSUMI Toshifumi, Tokyo 113-8654 (JP); KODAMA Tatsuhiko, Tokyo 113-8654 (JP); SUGIYAMA Akira, Tokyo 113-8654 (JP); TSUKAGOSHI Masanobu, Tokyo 168-0064 (JP)
(74) Representative: Godemeyer Blum Lenze Patentanwälte Partnerschaft mbB - werkpatent
(86) International application number: PCT/JP2019/021586
(87) International publication number: WO 2019/230905

(57) **Abstract**

An object of the present invention is to provide a halogenated biotin-modified dimer, which is capable of imaging diagnosis or treatment by labeling with a halogen, a biotin-modified dimer having a high affinity for a mutant streptavidin with a low affinity for natural biotin. The present invention provides a compound represented by the following formula (1) or a salt thereof: 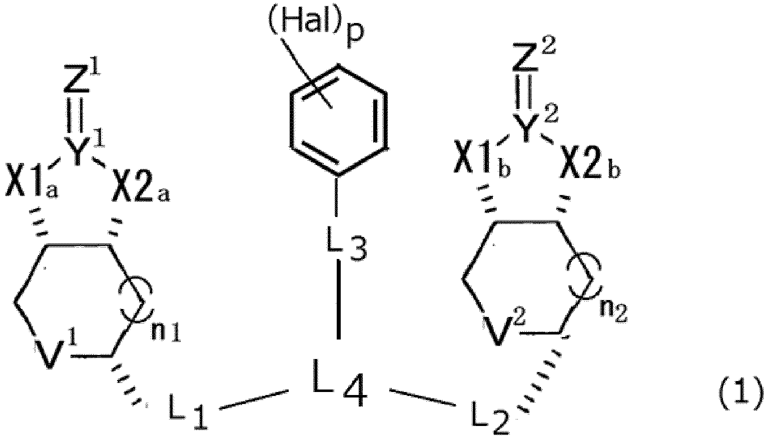 wherein the meaning of each symbol is the same as that described in the specification.

SEQUENCE LISTING
<110>  Tokyo University
<110>  Savid Therapeutics Inc
<120>  halogenized biotin-modified dimer and use thereof
<130>  191494A
<160>  19
<210>  1
<211>  133
<212>  PRT
<213> Artificial Sequence
<220>
<223> Description of Artificial Sequence: Recombinant protein
<400>  1
Ala Glu Ala Gly Ile Thr Gly Thr Trp Ser Asp Gln Leu Gly Asp Thr
1               5                   10                  15
Phe Ile Val Thr Ala Gly Ala Asp Gly Ala Leu Thr Gly Thr Tyr Glu
            20                  25                  30
Asn Ala Val Gly Gly Ala Glu Ser Arg Tyr Val Leu Thr Gly Arg Tyr
        35                  40                  45
Asp Ser Ala Pro Ala Thr Asp Gly Ser Gly Thr Ala Leu Gly Trp Thr
    50                  55                  60
Val Ala Trp Lys Asn Asn Ser Lys Asn Ala His Ser Ala Thr Thr Trp
65                  70                  75                  80
Ser Gly Gln Tyr Val Gly Gly Ala Asp Ala Lys Ile Asn Thr Gln Trp
                85                  90                  95
Leu Leu Thr Ser Gly Thr Thr Asn Ala Asn Ala Trp Lys Ser Thr Leu
            100                 105                 110
Val Gly His Asp Thr Phe Thr Lys Val Lys Pro Ser Ala Ala Ser His
        115                 120                 125
His His His His His
    130
<210>  2
<211>  241
<212>  PRT
<213> Artificial Sequence
<220>
<223> Description of Artificial Sequence: Recombinant protein
<400>  2
Gln Val Lys Leu Glu Gln Ser Gly Ala Glu Val Val Lys Pro Gly Ala
1               5                   10                  15
Ser Val Lys Leu Ser Cys Lys Ala Ser Gly Phe Asn Ile Lys Asp Ser
            20                  25                  30
Tyr Met His Trp Leu Arg Gln Gly Pro Gly Gln Arg Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ala Asn Thr Ala Tyr
65                  70                  75                  80
Leu Gly Leu Ser Ser Leu Arg Pro Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ser
    130                 135                 140
Ser Met Ser Val Ser Val Gly Asp Arg Val Asn Ile Ala Cys Ser Ala
145                 150                 155                 160
Ser Ser Ser Val Pro Tyr Met His Trp Leu Gln Gln Lys Pro Gly Lys
                165                 170                 175
Ser Pro Lys Leu Leu Ile Tyr Leu Thr Ser Asn Leu Ala Ser Gly Val
            180                 185                 190
Pro Ser Arg Phe Ser Gly Ser Gly Ser Gly Thr Asp Tyr Ser Leu Thr

195                 200                 205
Ile Ser Ser Val Gln Pro Glu Asp Ala Ala Thr Tyr Tyr Cys Gln Gln
    210                 215                 220
Arg Ser Ser Tyr Pro Leu Thr Phe Gly Gly Gly Thr Lys Leu Glu Ile
225                 230                 235                 240
Lys

<210>  3
<211>  383
<212>  PRT
<213> Artificial Sequence
<220>
<223> Description of Artificial Sequence: Recombinant protein
<400>  3
Gln Val Lys Leu Glu Gln Ser Gly Ala Glu Val Val Lys Pro Gly Ala
1               5                   10                  15
Ser Val Lys Leu Ser Cys Lys Ala Ser Gly Phe Asn Ile Lys Asp Ser
            20                  25                  30
Tyr Met His Trp Leu Arg Gln Gly Pro Gly Gln Arg Leu Glu Trp Ile
        35                  40                  45
Gly Trp Ile Asp Pro Glu Asn Gly Asp Thr Glu Tyr Ala Pro Lys Phe
    50                  55                  60
Gln Gly Lys Ala Thr Phe Thr Thr Asp Thr Ser Ala Asn Thr Ala Tyr
65                  70                  75                  80
Leu Gly Leu Ser Ser Leu Arg Pro Glu Asp Thr Ala Val Tyr Tyr Cys
                85                  90                  95
Asn Glu Gly Thr Pro Thr Gly Pro Tyr Tyr Phe Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Glu Asn Val Leu Thr Gln Ser Pro Ser
    130                 135                 140
Ser Met Ser Val Ser Val Gly Asp Arg Val Asn Ile Ala Cys Ser Ala
145                 150                 155                 160
Ser Ser Ser Val Pro Tyr Met His Trp Leu Gln Gln Lys Pro Gly Lys
                165                 170                 175
Ser Pro Lys Leu Leu Ile Tyr Leu Thr Ser Asn Leu Ala Ser Gly Val
            180                 185                 190
Pro Ser Arg Phe Ser Gly Ser Gly Ser Gly Thr Asp Tyr Ser Leu Thr
        195                 200                 205
Ile Ser Ser Val Gln Pro Glu Asp Ala Ala Thr Tyr Tyr Cys Gln Gln
    210                 215                 220
Arg Ser Ser Tyr Pro Leu Thr Phe Gly Gly Gly Thr Lys Leu Glu Ile
225                 230                 235                 240
Lys Gly Gly Gly Gly Ser Gly Gly Gly Gly Ala Glu Ala Gly Ile Thr
                245                 250                 255
Gly Thr Trp Ser Asp Gln Leu Gly Asp Thr Phe Ile Val Thr Ala Gly
            260                 265                 270
Ala Asp Gly Ala Leu Thr Gly Thr Tyr Glu Asn Ala Val Gly Gly Ala
        275                 280                 285
Glu Ser Arg Tyr Val Leu Thr Gly Arg Tyr Asp Ser Ala Pro Ala Thr
    290                 295                 300
Asp Gly Ser Gly Thr Ala Leu Gly Trp Thr Val Ala Trp Lys Asn Asn
305                 310                 315                 320
Ser Lys Asn Ala His Ser Ala Thr Thr Trp Ser Gly Gln Tyr Val Gly
                325                 330                 335
Gly Ala Asp Ala Lys Ile Asn Thr Gln Trp Leu Leu Thr Ser Gly Thr
            340                 345                 350
Thr Asn Ala Asn Ala Trp Lys Ser Thr Leu Val Gly His Asp Thr Phe
        355                 360                 365
Thr Lys Val Lys Pro Ser Ala Ala Ser His His His His His His
    370                 375                 380
<210>  4

<211>  405
<212>  PRT
<213> Artificial Sequence
<220>
<223> Description of Artificial Sequence: Recombinant protein
<400>  4
Met Lys Tyr Leu Leu Pro Thr Ala Ala Ala Gly Leu Leu Leu Leu Ala
1               5                   10                  15
Ala Gln Pro Ala Met Ala Gln Val Lys Leu Glu Gln Ser Gly Ala Glu
            20                  25                  30
Val Val Lys Pro Gly Ala Ser Val Lys Leu Ser Cys Lys Ala Ser Gly
        35                  40                  45
Phe Asn Ile Lys Asp Ser Tyr Met His Trp Leu Arg Gln Gly Pro Gly
    50                  55                  60
Gln Arg Leu Glu Trp Ile Gly Trp Ile Asp Pro Glu Asn Gly Asp Thr
65                  70                  75                  80
Glu Tyr Ala Pro Lys Phe Gln Gly Lys Ala Thr Phe Thr Thr Asp Thr
                85                  90                  95
Ser Ala Asn Thr Ala Tyr Leu Gly Leu Ser Ser Leu Arg Pro Glu Asp
            100                 105                 110
Thr Ala Val Tyr Tyr Cys Asn Glu Gly Thr Pro Thr Gly Pro Tyr Tyr
        115                 120                 125
Phe Asp Tyr Trp Gly Gln Gly Thr Leu Val Thr Val Ser Ser Gly Gly
    130                 135                 140
Gly Gly Ser Gly Gly Gly Gly Ser Gly Gly Gly Gly Ser Glu Asn Val
145                 150                 155                 160
Leu Thr Gln Ser Pro Ser Ser Met Ser Val Ser Val Gly Asp Arg Val
                165                 170                 175
Asn Ile Ala Cys Ser Ala Ser Ser Ser Val Pro Tyr Met His Trp Leu
            180                 185                 190
Gln Gln Lys Pro Gly Lys Ser Pro Lys Leu Leu Ile Tyr Leu Thr Ser
        195                 200                 205
Asn Leu Ala Ser Gly Val Pro Ser Arg Phe Ser Gly Ser Gly Ser Gly
    210                 215                 220
Thr Asp Tyr Ser Leu Thr Ile Ser Ser Val Gln Pro Glu Asp Ala Ala
225                 230                 235                 240
Thr Tyr Tyr Cys Gln Gln Arg Ser Ser Tyr Pro Leu Thr Phe Gly Gly
                245                 250                 255
Gly Thr Lys Leu Glu Ile Lys Gly Gly Gly Gly Ser Gly Gly Gly Gly
            260                 265                 270
Ala Glu Ala Gly Ile Thr Gly Thr Trp Ser Asp Gln Leu Gly Asp Thr
        275                 280                 285
Phe Ile Val Thr Ala Gly Ala Asp Gly Ala Leu Thr Gly Thr Tyr Glu
    290                 295                 300
Asn Ala Val Gly Gly Ala Glu Ser Arg Tyr Val Leu Thr Gly Arg Tyr
305                 310                 315                 320
Asp Ser Ala Pro Ala Thr Asp Gly Ser Gly Thr Ala Leu Gly Trp Thr
                325                 330                 335
Val Ala Trp Lys Asn Asn Ser Lys Asn Ala His Ser Ala Thr Thr Trp
            340                 345                 350
Ser Gly Gln Tyr Val Gly Gly Ala Asp Ala Lys Ile Asn Thr Gln Trp
        355                 360                 365
Leu Leu Thr Ser Gly Thr Thr Asn Ala Asn Ala Trp Lys Ser Thr Leu
    370                 375                 380
Val Gly His Asp Thr Phe Thr Lys Val Lys Pro Ser Ala Ala Ser His
385                 390                 395                 400
His His His His His
                405
<210>  5
<211>  1218
<212>  DNA
<213> Artificial Sequence
<220>

<223> Description of Artificial Sequence: Recombinant DNA
<400> 5
atgaaatatc tgctgccgac cgcagcagcg ggtctgctgc tgctggcagc acagcctgca 60
atggcacagg ttaaactgga acagagcggt gccgaagttg ttaaaccggg tgcaagcgtt 120
aaactgagct gtaaagcaag cggctttaac atcaaagata gctatatgca ttggctgcgt 180
cagggtccgg gtcagcgtct ggaatggatt ggttggattg atccggaaaa tggtgatacc 240
gaatatgcac cgaaatttca gggtaaagca acctttacca ccgataccag cgcaaatacc 300
gcatatctgg gtctgagcag cctgcgtccg gaagataccg cagtgtatta ttgtaatgaa 360
ggcaccccga ccggtccgta ttatttcgat tattggggtc agggcaccct ggttaccgtt 420
agcagcggtg gtggtggtag tggtggcggt ggttcaggcg gtggcggtag cgaaaatgtt 480
ctgacccaga gcccgagcag catgagcgtt agcgttggtg atcgtgttaa tattgcatgt 540
agcgcaagca gcagcgttcc gtacatgcac tggctgcagc agaaaccggg taaaagcccg 600
aaactgctga tttatctgac cagcaatctg gcaagcggtg ttccgagccg ttttagcggt 660
agcggtagtg gcaccgatta tagcctgacc attagcagcg tgcagcctga agatgcagca 720
acctattatt gtcagcagcg tagcagttat ccgctgacct ttggtggtgg caccaaactg 780
gaaattaaag ggggtggtgg ctcaggtggc ggaggtgcag aagcaggtat taccggtaca 840
tggtcagatc agctgggtga tacctttatt gttaccgcag gcgcagatgg tgcactgacc 900
ggcacctatg aaaatgcagt tggtggtgca gaaagccgtt atgtgctgac cggtcgttat 960
gatagcgcac cggcaaccga tggtagcggc accgcactgg gttggaccgt tgcatggaaa 1020
aataacagca aaaatgcaca tagcgcaacc acctggtcag gtcagtatgt gggtggtgcc 1080
gatgccaaaa ttaacaccca gtggctgctg accagcggta caaccaatgc aaatgcctgg 1140
aaaagtaccc tggttggtca tgatacattc accaaagtta aaccgagcgc agcaagccat 1200
catcatcacc atcattaa 1218
<210> 6
<211> 141
<212> PRT
<213> Artificial Sequence
<220>
<223> Description of Artificial Sequence: Recombinant protein
<400> 6
Met Asp Lys Ile Ala Ile Val Asn Met Gly Ser Leu Phe Gln Gln Val
1               5                   10                  15
Ala Gln Lys Thr Gly Val Ser Asn Thr Leu Glu Asn Glu Phe Lys Gly
            20                  25                  30
Arg Ala Ser Glu Leu Gln Arg Met Glu Thr Asp Leu Gln Ala Lys Met
        35                  40                  45
Lys Lys Leu Gln Ser Met Lys Ala Gly Ser Asp Arg Thr Lys Leu Glu
    50                  55                  60
Lys Asp Val Met Ala Gln Arg Gln Thr Phe Ala Gln Lys Ala Gln Ala
65                  70                  75                  80
Phe Glu Gln Asp Arg Ala Arg Arg Ser Asn Glu Glu Arg Gly Lys Leu
                85                  90                  95
Val Thr Arg Ile Gln Thr Ala Val Lys Ser Val Ala Asn Ser Gln Asp
            100                 105                 110
Ile Asp Leu Val Val Asp Ala Asn Ala Val Ala Tyr Asn Ser Ser Asp
        115                 120                 125
Val Lys Asp Ile Thr Ala Asp Val Leu Lys Gln Val Lys
    130                 135                 140
<210> 7
<211> 247
<212> PRT
<213> Artificial Sequence
<220>
<223> Description of Artificial Sequence: Recombinant protein
<400> 7
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45
Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Gly Gly Gly Gly Ser Asp Ile Gln Met
    130                 135                 140
Thr Gln Ser Pro Ser Ser Leu Ser Ala Ser Val Gly Asp Arg Val Thr
145                 150                 155                 160
Ile Thr Cys Arg Ala Ser Gln Asp Val Asn Thr Ala Val Ala Trp Tyr
                165                 170                 175
Gln Gln Lys Pro Gly Lys Ala Pro Lys Leu Leu Ile Tyr Ser Ala Ser
            180                 185                 190
Phe Leu Tyr Ser Gly Val Pro Ser Arg Phe Ser Gly Ser Arg Ser Gly
        195                 200                 205
Thr Asp Phe Thr Leu Thr Ile Ser Ser Leu Gln Pro Glu Asp Phe Ala
    210                 215                 220
Thr Tyr Tyr Cys Gln Gln His Tyr Thr Thr Pro Pro Thr Phe Gly Gln
225                 230                 235                 240
Gly Thr Lys Val Glu Ile Lys
                245
<210>  8
<211>  391
<212>  PRT
<213> Artificial Sequence
<220>
<223> Description of Artificial Sequence: Recombinant protein
<400>  8
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45
Ala Arg Ile Tyr Pro Thr Asn Gly Tyr Thr Arg Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Gly Gly Gly Gly Ser Gly Gly Gly
        115                 120                 125
Gly Ser Gly Gly Gly Gly Ser Gly Gly Gly Gly Ser Asp Ile Gln Met
    130                 135                 140
Thr Gln Ser Pro Ser Ser Leu Ser Ala Ser Val Gly Asp Arg Val Thr
145                 150                 155                 160
Ile Thr Cys Arg Ala Ser Gln Asp Val Asn Thr Ala Val Ala Trp Tyr
                165                 170                 175
Gln Gln Lys Pro Gly Lys Ala Pro Lys Leu Leu Ile Tyr Ser Ala Ser
            180                 185                 190
Phe Leu Tyr Ser Gly Val Pro Ser Arg Phe Ser Gly Ser Arg Ser Gly
        195                 200                 205
Thr Asp Phe Thr Leu Thr Ile Ser Ser Leu Gln Pro Glu Asp Phe Ala
    210                 215                 220
Thr Tyr Tyr Cys Gln Gln His Tyr Thr Thr Pro Pro Thr Phe Gly Gln
225                 230                 235                 240
Gly Thr Lys Val Glu Ile Lys Gly Gly Gly Gly Gly Ser Gly Gly Gly
                245                 250                 255

Gly Gly Ala Glu Ala Gly Ile Thr Gly Thr Trp Ser Asp Gln Leu Gly
                260                 265                 270
Asp Thr Phe Ile Val Thr Ala Gly Ala Asp Gly Ala Leu Thr Gly Thr
            275                 280                 285
Tyr Glu Asn Ala Val Gly Gly Ala Glu Ser Arg Tyr Val Leu Thr Gly
        290                 295                 300
Arg Tyr Asp Ser Ala Pro Ala Thr Asp Gly Ser Gly Thr Ala Leu Gly
305                 310                 315                 320
Trp Thr Val Ala Trp Lys Asn Asn Ser Lys Asn Ala His Ser Ala Thr
                325                 330                 335
Thr Trp Ser Gly Gln Tyr Val Gly Gly Ala Asp Ala Lys Ile Asn Thr
            340                 345                 350
Gln Trp Leu Leu Thr Ser Gly Thr Thr Asn Ala Asn Ala Trp Lys Ser
        355                 360                 365
Thr Leu Val Gly His Asp Thr Phe Thr Lys Val Lys Pro Ser Ala Ala
    370                 375                 380
Ser His His His His His His
385                 390
<210>  9
<211>  413
<212>  PRT
<213> Artificial Sequence
<220>
<223> Description of Artificial Sequence: Recombinant protein
<400>  9
Met Lys Tyr Leu Leu Pro Thr Ala Ala Ala Gly Leu Leu Leu Leu Ala
1               5                   10                  15
Ala Gln Pro Ala Met Ala Glu Val Gln Leu Val Glu Ser Gly Gly Gly
            20                  25                  30
Leu Val Gln Pro Gly Gly Ser Leu Arg Leu Ser Cys Ala Ala Ser Gly
        35                  40                  45
Phe Asn Ile Lys Asp Thr Tyr Ile His Trp Val Arg Gln Ala Pro Gly
    50                  55                  60
Lys Gly Leu Glu Trp Val Ala Arg Ile Tyr Pro Thr Asn Gly Tyr Thr
65                  70                  75                  80
Arg Tyr Ala Asp Ser Val Lys Gly Arg Phe Thr Ile Ser Ala Asp Thr
                85                  90                  95
Ser Lys Asn Thr Ala Tyr Leu Gln Met Asn Ser Leu Arg Ala Glu Asp
            100                 105                 110
Thr Ala Val Tyr Tyr Cys Ser Arg Trp Gly Gly Asp Gly Phe Tyr Ala
        115                 120                 125
Met Asp Tyr Trp Gly Gln Gly Thr Leu Val Thr Val Ser Ser Gly Gly
    130                 135                 140
Gly Gly Ser Gly Gly Gly Gly Ser Gly Gly Gly Gly Ser Gly Gly Gly
145                 150                 155                 160
Gly Ser Asp Ile Gln Met Thr Gln Ser Pro Ser Ser Leu Ser Ala Ser
                165                 170                 175
Val Gly Asp Arg Val Thr Ile Thr Cys Arg Ala Ser Gln Asp Val Asn
            180                 185                 190
Thr Ala Val Ala Trp Tyr Gln Gln Lys Pro Gly Lys Ala Pro Lys Leu
        195                 200                 205
Leu Ile Tyr Ser Ala Ser Phe Leu Tyr Ser Gly Val Pro Ser Arg Phe
    210                 215                 220
Ser Gly Ser Arg Ser Gly Thr Asp Phe Thr Leu Thr Ile Ser Ser Leu
225                 230                 235                 240
Gln Pro Glu Asp Phe Ala Thr Tyr Tyr Cys Gln Gln His Tyr Thr Thr
                245                 250                 255
Pro Pro Thr Phe Gly Gln Gly Thr Lys Val Glu Ile Lys Gly Gly Gly
            260                 265                 270
Gly Gly Ser Gly Gly Gly Gly Gly Ala Glu Ala Gly Ile Thr Gly Thr
        275                 280                 285
Trp Ser Asp Gln Leu Gly Asp Thr Phe Ile Val Thr Ala Gly Ala Asp
    290                 295                 300

Gly Ala Leu Thr Gly Thr Tyr Glu Asn Ala Val Gly Gly Ala Glu Ser
305                 310                 315                 320
Arg Tyr Val Leu Thr Gly Arg Tyr Asp Ser Ala Pro Ala Thr Asp Gly
                325                 330                 335
Ser Gly Thr Ala Leu Gly Trp Thr Val Ala Trp Lys Asn Asn Ser Lys
            340                 345                 350
Asn Ala His Ser Ala Thr Thr Trp Ser Gly Gln Tyr Val Gly Gly Ala
        355                 360                 365
Asp Ala Lys Ile Asn Thr Gln Trp Leu Leu Thr Ser Gly Thr Thr Asn
    370                 375                 380
Ala Asn Ala Trp Lys Ser Thr Leu Val Gly His Asp Thr Phe Thr Lys
385                 390                 395                 400
Val Lys Pro Ser Ala Ala Ser His His His His His His
                405                 410
<210>  10
<211>  1242
<212>  DNA
<213> Artificial Sequence
<220>
<223> Description of Artificial Sequence: Recombinant DNA
<400>  10
atgaaatatc tgctgccgac cgcagcagcg ggtctgctgc tgctggcagc acagcctgca      60
atggcagaag ttcagctggt tgaaagcggt ggtggtctgg ttcagcctgg tggtagcctg     120
cgtctgagct gtgcagcaag cggttttaac attaaagata cctatattca ttgggtgcgt     180
caggcacctg gtaaaggtct ggaatgggtt gcacgtattt atccgaccaa tggttatacc     240
cgttatgccg atagcgttaa aggtcgtttt accattagcg cagataccag caaaaatacc     300
gcatacctgc agatgaatag tctgcgtgca gaggataccg cagtgtatta ttgtagccgt     360
tggggtggtg atggttttta tgcaatggat tattggggtc agggcaccct ggttaccgtt     420
agctcaggtg gaggcggttc cggtggcgga ggttccggtg gaggtggctc cggtggcgga     480
ggttccgata ttcagatgac ccagagtccg agcagcctga gcgcaagcgt tggtgatcgt     540
gtgaccatta cctgtcgtgc aagccaggat gttaatacag cagttgcatg gtatcagcag     600
aaaccgggta aagcaccgaa actgctgatt tatagcgcaa gctttctgta tagcggtgtt     660
ccgagccgtt ttagcggtag ccgtagcggc accgatttta ccctgaccat tagcagcctg     720
cagccggaag attttgcaac ctattattgt cagcagcatt acaccacacc gcctaccttt     780
ggccagggca ccaaagttga aattaaagga ggtggcggtg gatccggcgg aggtggcgga     840
gcagaagcag gtattaccgg tacatggtca gatcagctgg gtgatacctt tattgttacc     900
gcaggcgcag atggtgcact gaccggcacc tatgaaaatg cagttggtgg tgcagaaagc     960
cgttatgtgc tgaccggtcg ttatgatagc gcaccggcaa ccgatggtag cggcaccgca    1020
ctgggttgga ccgttgcatg gaaaaataac agcaaaaatg cacatagcgc aaccacctgg    1080
tcaggtcagt atgtgggtgg tgccgatgcc aaaattaaca cccagtggct gctgaccagc    1140
ggtacaacca atgcaaatgc ctggaaaagt accctggttg gtcatgatac attcaccaaa    1200
gttaaaccga gcgcagcaag ccatcatcat caccatcatt aa                       1242
<210>  11
<211>  9
<212>  PRT
<213> Artificial Sequence
<220>
<223> Description of Artificial Sequence: Recombinant linker
<400>  11
Gly Gly Gly Gly Ser Gly Gly Gly Gly
1               5
<210>  12
<211>  40
<212>  DNA
<213> Artificial Sequence
<220>
<223> Description of Artificial Sequence: Synthesized DNA
<400>  12
aaggagatat acatatggat aaaattgcca ttgttaatat                           40
<210>  13
<211>  39
<212>  DNA
<213> Artificial Sequence

<220>
<223> Description of Artificial Sequence: Synthesized DNA
<400> 13
ttgagatctg ccatatgtta tttcacttgt ttcagaacg 39
<210> 14
<211> 35
<212> DNA
<213> Artificial Sequence
<220>
<223> Description of Artificial Sequence: Synthesized DNA
<400> 14
agaaggagat ataccatgaa atatctgctg ccgac 35
<210> 15
<211> 36
<212> DNA
<213> Artificial Sequence
<220>
<223> Description of Artificial Sequence: Synthesized DNA
<400> 15
cgccgagctc gaattttaat gatggtgatg atgatg 36
<210> 16
<211> 26
<212> DNA
<213> Artificial Sequence
<220>
<223> Description of Artificial Sequence: Synthesized DNA
<400> 16
ggtatatctc cttcttaaag ttaaac 26
<210> 17
<211> 24
<212> DNA
<213> Artificial Sequence
<220>
<223> Description of Artificial Sequence: Synthesized DNA
<400> 17
aattcgagct cggcgcgcct gcag 24
<210> 18
<211> 11
<212> PRT
<213> Artificial Sequence
<220>
<223> Description of Artificial Sequence: Recombinant linker
<400> 18
Gly Gly Gly Gly Gly Ser Gly Gly Gly Gly Gly
1               5                   10
<210> 19
<211> 127
<212> PRT
<213> Artificial Sequence
<220>
<223> Description of Artificial Sequence: Recombinant peptide
<400> 19
Ala Glu Ala Gly Ile Thr Gly Thr Trp Ser Asp Gln Leu Gly Asp Thr
1               5                   10                  15
Phe Ile Val Thr Ala Gly Ala Asp Gly Ala Leu Thr Gly Thr Tyr Glu
            20                  25                  30
Asn Ala Val Gly Gly Ala Glu Ser Arg Tyr Val Leu Thr Gly Arg Tyr
        35                  40                  45
Asp Ser Ala Pro Ala Thr Asp Gly Ser Gly Thr Ala Leu Gly Trp Thr
    50                  55                  60
Val Ala Trp Lys Asn Asn Ser Lys Asn Ala His Ser Ala Thr Thr Trp
65                  70                  75                  80
Ser Gly Gln Tyr Val Gly Gly Ala Asp Ala Lys Ile Asn Thr Gln Trp

                85                  90                  95
Leu Leu Thr Ser Gly Thr Thr Asn Ala Asn Ala Trp Lys Ser Thr Leu
            100                 105                 110
Val Gly His Asp Thr Phe Thr Lys Val Lys Pro Ser Ala Ala Ser
        115                 120                 125

## Description

### Technical Field

The present invention relates to a halogenated biotin-modified dimer and the use thereof.

### Background Art

Avidin and biotin, or streptavidin and biotin have an extremely high affinity between them (Kd = 10⁻¹⁵ to 10⁻¹⁴ M). This is one of the strongest interactions between two biomolecules. At present, the interaction between avidin/streptavidin and biotin has been widely applied in the field of biochemistry, molecular biology, or medicine. A drug delivery method in which high binding ability between avidin/streptavidin and biotin is combined with an antibody molecule, namely, a pretargeting method has been devised.

Since chicken-derived avidin or microorganism-derived streptavidin exhibits high immunogenicity to a human body, such avidin or streptavidin is problematic in that an anti-avidin/streptavidin antibody is generated in an early stage after the administration thereof to a human body. This causes prevention of the practical use of the pretargeting method. Low immunogenic streptavidin that solves the aforementioned problem has been reported (International Publication WO2010/095455).

Low immunogenic streptavidin is characterized in that its immunogenicity to a human body is reduced. Since the low immunogenic streptavidin has an affinity for biotin existing in a human body, the low immunogenic streptavidin has been problematic in that it causes high background when used for diagnoses, or in that it is not likely to exhibit medicinal effects specifically on a disease when used for treatments.

Under such circumstances, a mutant streptavidin with a reduced affinity for natural biotin, and a modified biotin having a high affinity for the mutant streptavidin with a reduced affinity for natural biotin have been reported (International Publication WO2014/129446). Moreover, a mutant streptavidin with a reduced affinity for natural biotin, and a biotin-modified dimer having a high affinity for the mutant streptavidin with a low affinity for natural biotin have been reported (International Publication WO2015/125820).

### Prior Art Documents

### Patent Documents

Patent Document 1: International Publication WO2010/095455
Patent Document 2: International Publication WO2014/129446
Patent Document 3: International Publication WO2015/125820

### Summary of Invention

### Object to be Solved by the Invention

It is an object of the present invention to provide a halogenated biotin-modified dimer, which is capable of imaging diagnosis or treatment by labeling with a halogen, a biotin-modified dimer having a high affinity for a mutant streptavidin with a low affinity for natural biotin. Furthermore, it is another object of the present invention to provide a diagnostic kit and/or a therapeutic kit, in which a combination of the above-described halogenated biotin-modified dimer and a mutant streptavidin is used.

### Means for Solving the Object

The present inventor has conducted intensive studies directed towards achieving the aforementioned objects. The inventor has succeeded in introducing a halogen into the biotin-modified dimer described in International Publication WO2015/125820. Then, the present inventor has confirmed that the obtained halogenated biotin-modified dimer binds, with a high affinity, to a mutant streptavidin having a low affinity for a natural biotin, thereby completing the present invention.

Specifically, the present invention provides the following inventions.
[1] A compound represented by the following formula (1) or a salt thereof: 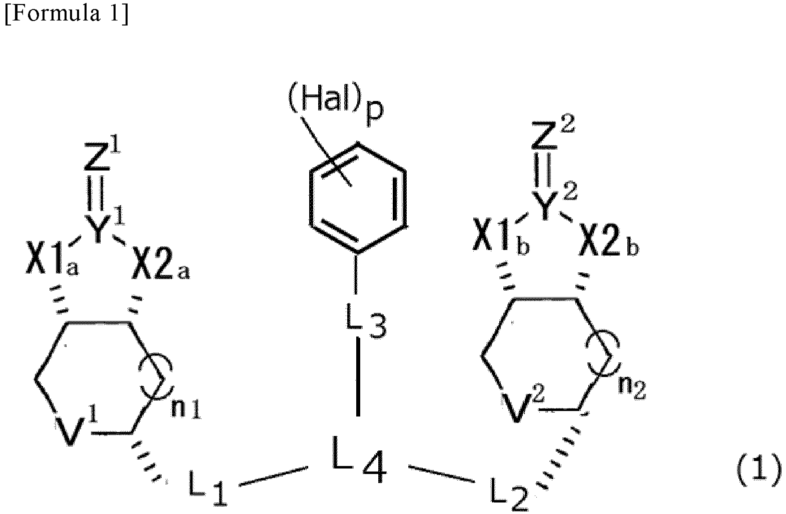 wherein
   X1a, X1b, X2a and X2b each independently represent O or NH,
   Y¹ and Y² each independently represent C or S,
   Z¹ and Z² each independently represent O, S or NH,
   V¹ and V² each independently represent S or S⁺-O⁻,
   n1 and n2 each independently represent an integer of 0 or 1,
   L₁, L₂, and L₃ each independently represent a divalent linking group,
   L₄ represents a trivalent linking group,
   Hal represents a halogen, and
   p represents an integer of 1 to 5.
[2] The compound according to the above [1], wherein the compound represented by the formula (1) is a compound represented by the following formula (1a) or the following formula (1b): 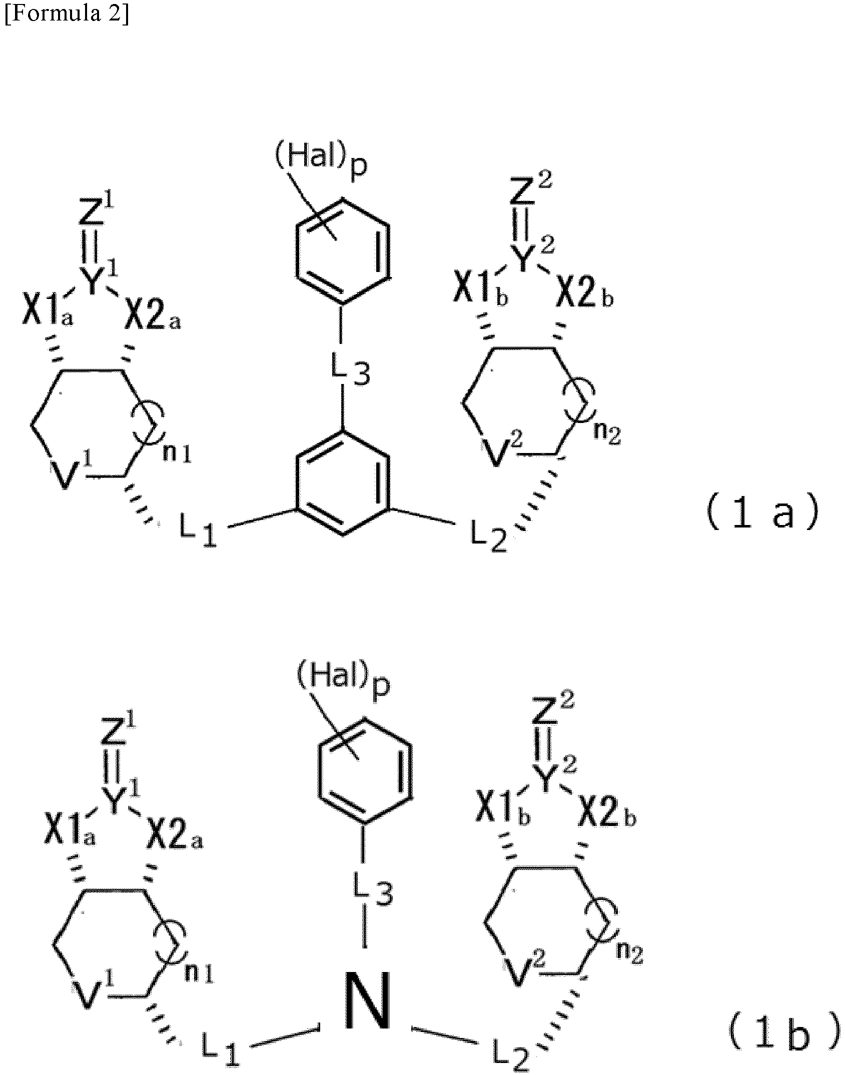 wherein each symbol is as defined in the above [1].
[3] The compound or a salt thereof according to the above [1] or [2], which is represented by the following formula (2) or the following formula (3), wherein n1 and n2 are 0. 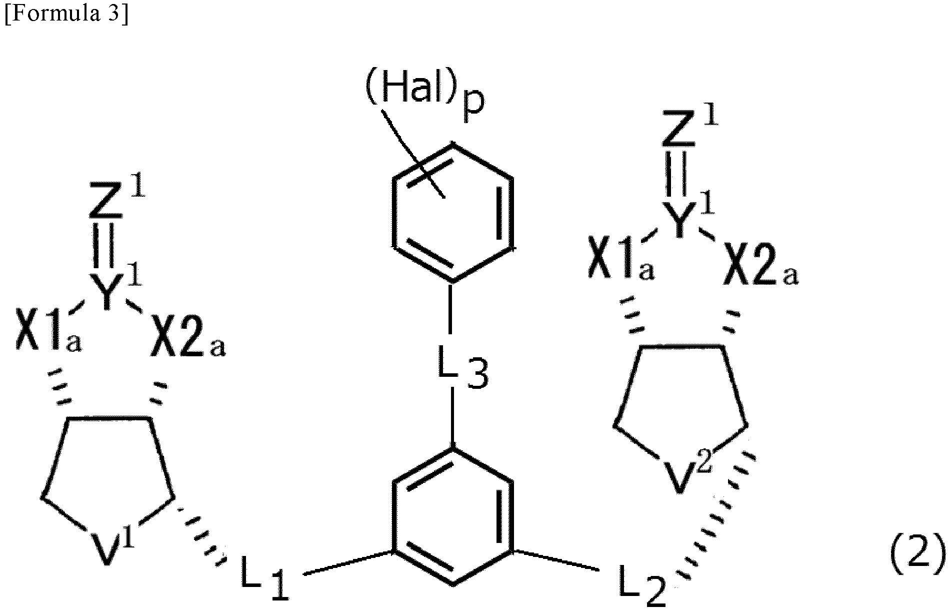 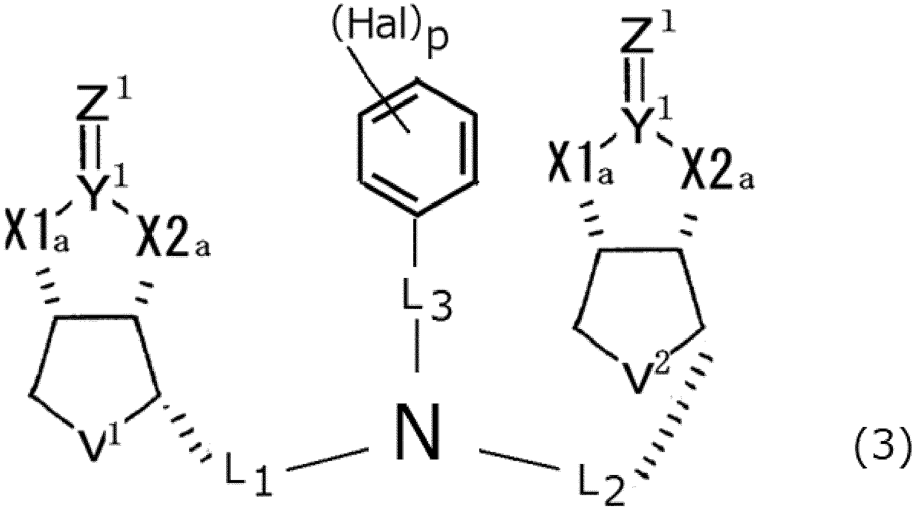 wherein each symbol is as defined in the above [1].
[4] The compound or a salt thereof according to any one of the above [1] to [3], wherein X1a, X1b, X2a and X2b represent NH, Y¹ and Y² represent C,
   Z¹ and Z² represent NH, and V¹ and V² represent S.
[5] The compound or a salt thereof according to any one of the above [1] to [4], wherein L₁, L₂, and L₃ each independently represent a divalent linking group consisting of a combination of groups selected from -CONH-, -NHCO-, -COO-, -OCO-, -CO-, -O-, and an alkylene group containing 1 to 10 carbon atoms.
[6] The compound or a salt thereof according to any one of the above [1] to [5], wherein
   L₁ represents -(CH₂)ₘ₁-L₁₁-(CH₂)ₘ₂-L₁₂-*,
   L₂ represents *-L₁₃-(CH₂)ₘ₃-L₁₄-(CH₂)ₘ₄-, and
   L3 represents L₁₅-L₂₁-L₁₆-,
   wherein L₁₁, L₁₂, L₁₃, L₁₄, L₁₅, and L₁₆ each independently represent -CONH-, -NHCO-, - COO-, -OCO-, -CO-, or -O-,
   m₁, m₂, m₃, and m₄ each independently represent an integer from 1 to 10,
   * represents a binding site with a benzene ring,
   L21 represents -CH₂-(OCH₂)ₘ₅-, and
   ms represents an integer from 1 to 10.
[7] The compound or a salt thereof according to any one of the above [1] to [6], wherein Hal represents I, ¹²⁵I, or ²¹¹At.
[8] The compound or a salt thereof according to any one of the above [1] to [7], wherein p is 1.
[9] A compound represented by any one of the following formulae: 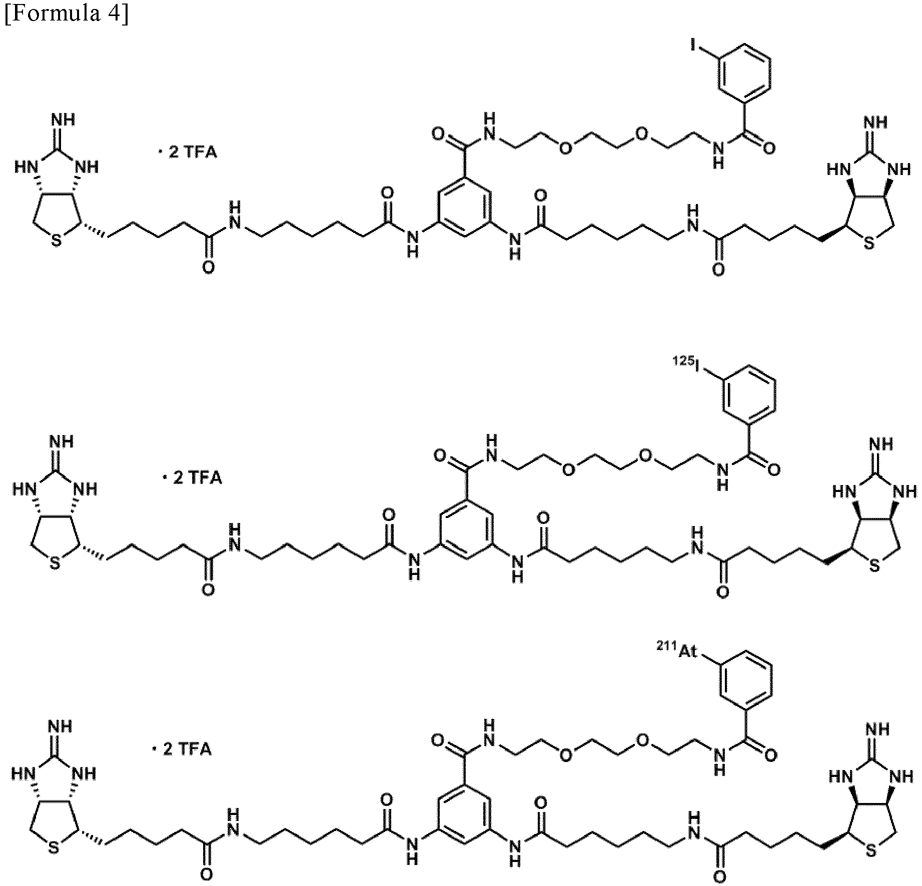 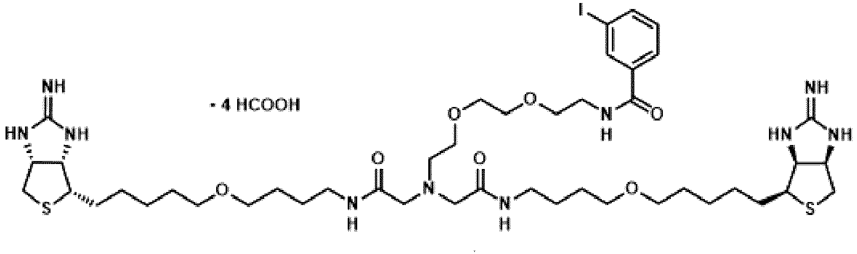
[10] A kit for treatment or diagnosis, comprising: (1) the compound according to any one of the above [1] to [9]; and (b) a mutant streptavidin-molecular probe conjugate obtained by binding a molecular probe to a mutant streptavidin comprising the amino acid sequence as set forth in SEQ ID NO: 19.
[11] A method for producing the compound or a salt thereof according to any one of the above [1] to [9], comprising reacting a compound represented by the following formula (A) with sodium halide or halogen: 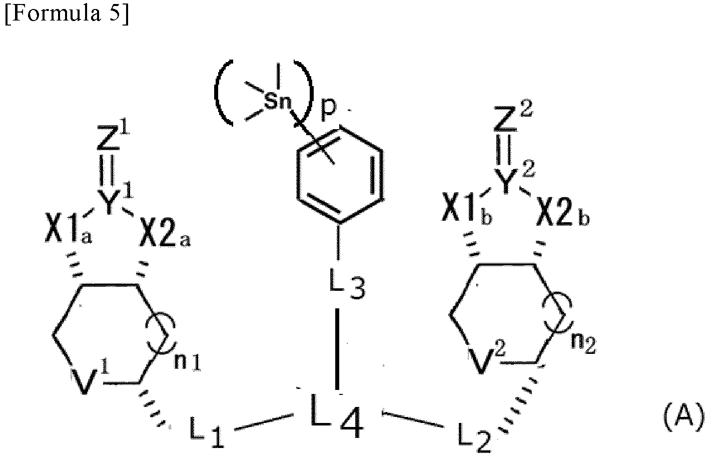 wherein each symbol is as defined in the above [1].
[12] A method for producing the compound or a salt thereof according to any one of the above [1] to [9], comprising reacting a compound represented by the following formula (B) with sodium halide or halogen: 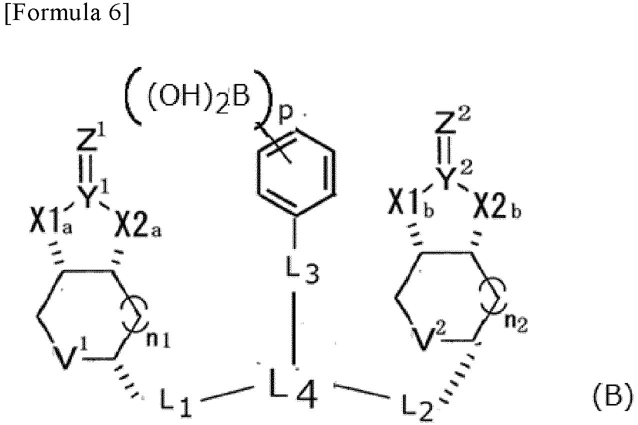 wherein each symbol is as defined in the above [1].

### Advantageous Effects of Invention

The halogenated biotin-modified dimer according to the present invention and a kit comprising the same are useful for diagnostic methods/therapeutic methods that are based on the pretargeting method.

### Brief Description of Drawings

[Fig. 1] Fig. 1 shows an outline of a domain structure.
[Fig. 2] Fig. 2 shows a CBB-stained SDS-PAGE electrophoretic pattern of CEA-V2122.
[Fig. 3] Fig. 3 shows a structural drawing of HER2-V2122.
[Fig. 4] Fig. 4 shows a CBB-stained SDS-PAGE electrophoretic pattern of HER2-V2122.
[Fig. 5] Fig. 5 shows the results obtained by confirming a binding conjugate of astatine-labeled Psyche B and Herceptin-Cupid, by using magnetic beads.
[Fig. 6] Fig. 6 shows the results obtained by confirming a binding conjugate of astatine-labeled Psyche B and Herceptin-Cupid, by using ovarian cancer cells SKOV3.

### Embodiment of Carrying out the Invention

Hereinafter, the present invention will be described in more detail.

### (1) Halogenated biotin-modified dimer

The present invention is a compound represented by the following formula (1) or a salt thereof, and is preferably a compound represented by the following formula (1a), the following formula (1b), the following formula (2) or the following formula (3) or a salt thereof. The compound of the present invention is also referred to as a "halogenated biotin-modified dimer." 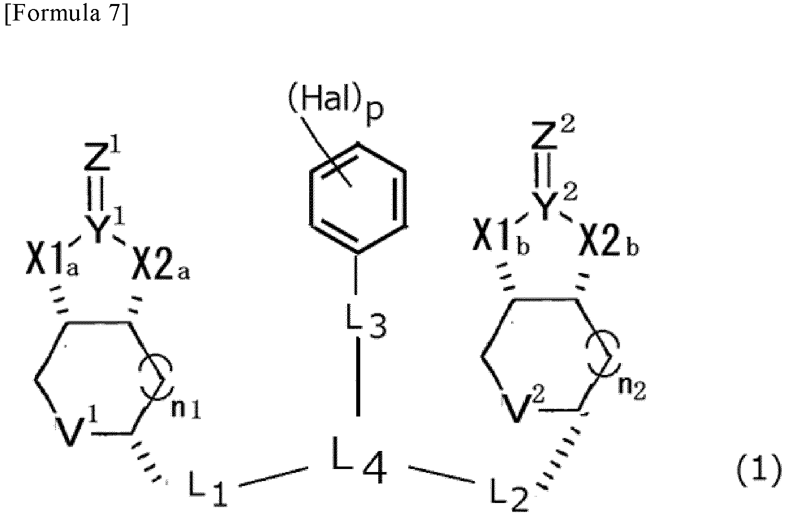 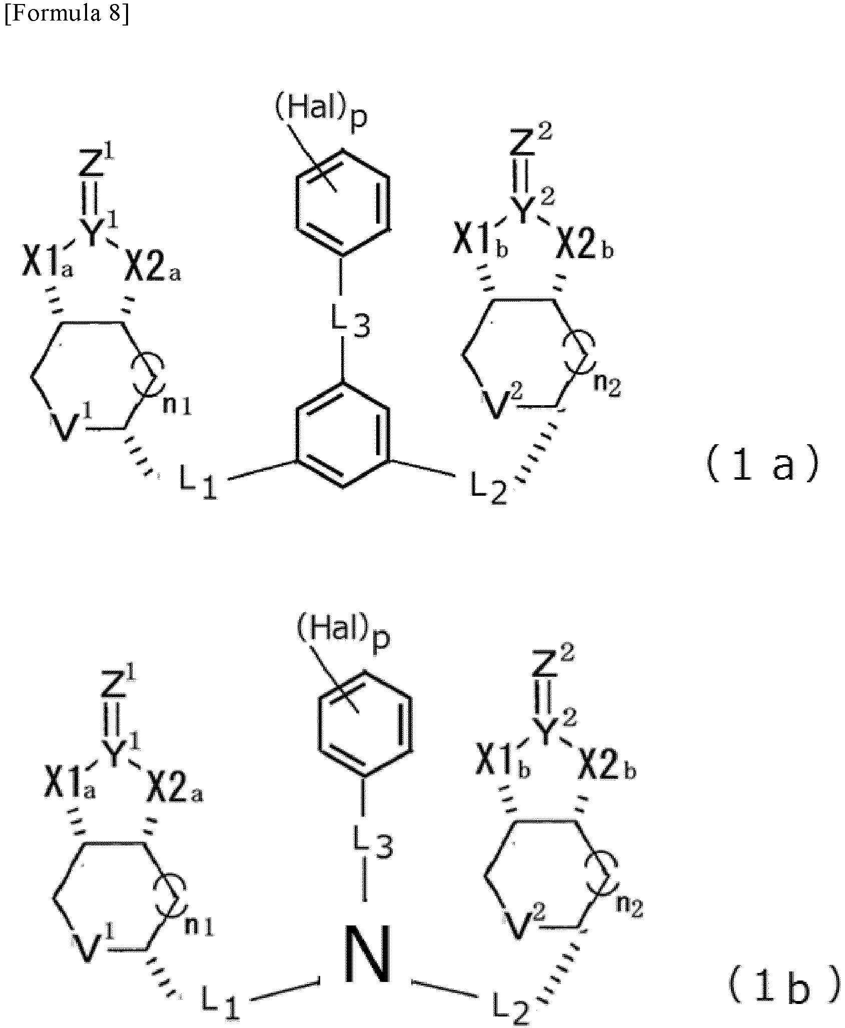 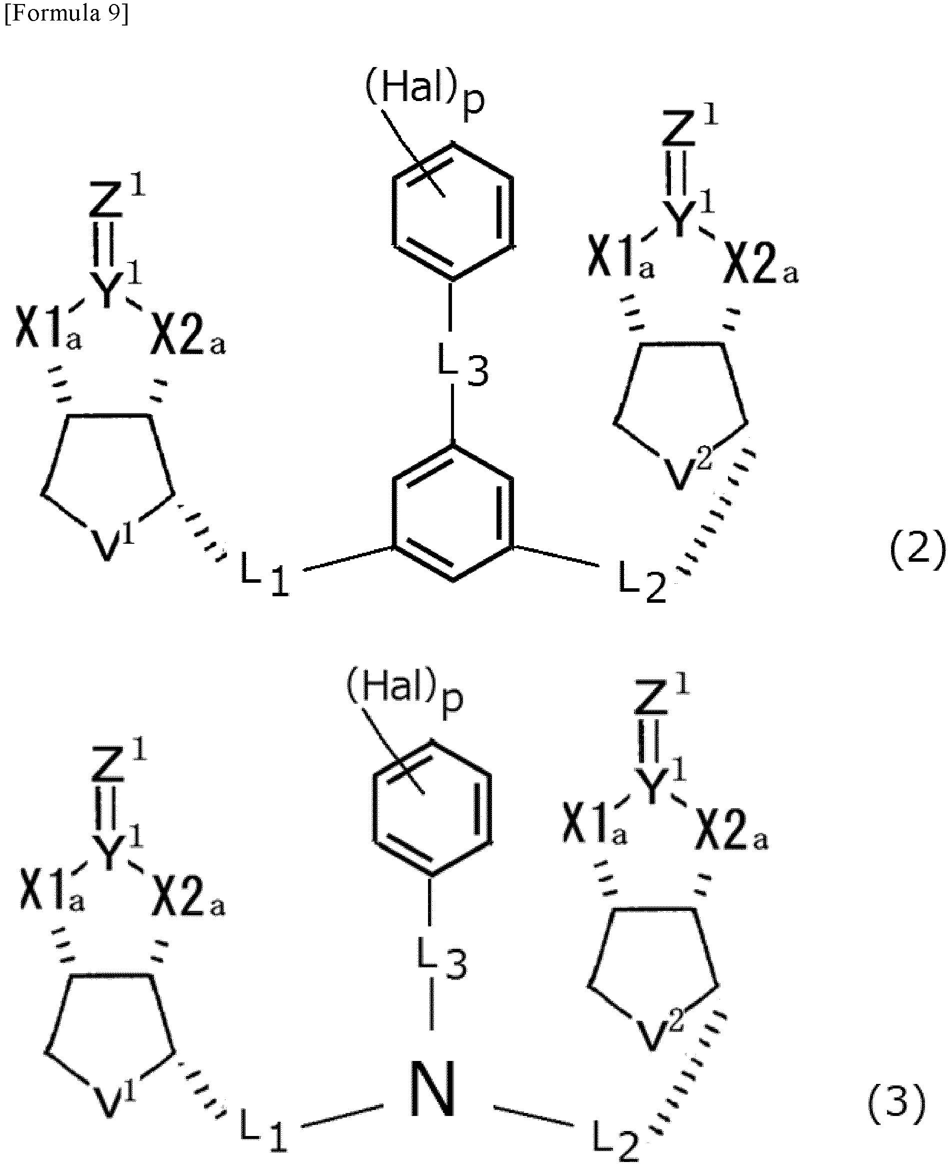 wherein
X1a, X1b, X2a and X2b each independently represent O or NH,
Y¹ and Y² each independently represent C or S,
Z¹ and Z² each independently represent O, S, or NH,
V¹ and V² each independently represent S or S⁺-O⁻,
n1 and n2 each independently represent an integer of 0 or 1,
L₁, L₂, and L₃ each independently represent a divalent linking group,
L₄ represents a trivalent linking group,
Hal represents a halogen, and
p represents an integer of 1 to 5.

In the formula (1), the formula (1a), the formula (1b), the formula (2), and the formula (3), the portions represented by the following structures: 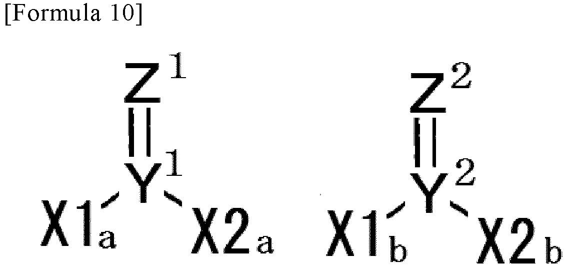 are preferably any one of the following portions, but are not limited thereto:

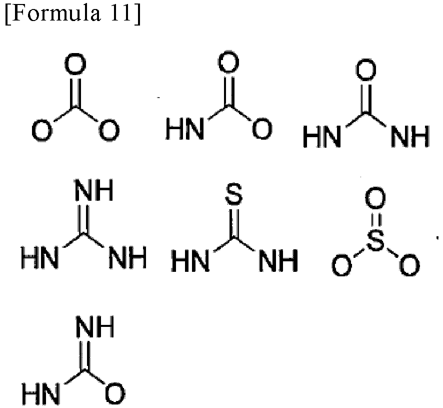

X1a, X1b, X2a and X2b preferably represent NH; Y¹ and Y² preferably represent C; Z¹ and Z² preferably represent NH; and V¹ and V² preferably represent S.

L₁, L₂, and L₃ each independently represent a divalent linking group consisting of a combination of groups selected from -CONH-, -NHCO-, -COO-, -OCO-, -CO-, -O-, and an alkylene group containing 1 to 10 carbon atoms.

L₁, L₂, and L₃ each independently represent a divalent linking group consisting of a combination of groups selected from -CONH-, -NHCO-, -O-, and an alkylene group containing 1 to 10 carbon atoms.

L₁ and L₂ each independently represent a divalent linking group consisting of a combination of groups selected from -CONH-, -NHCO-, and an alkylene group containing 1 to 10 carbon atoms.

L₁ preferably represents -(CH₂)ₘ₁-L₁₁-(CH₂)ₘ₂-L₁₂-*, L₂ preferably represents *-L₁₃-(CH₂)m₃-L₁₄-(CH₂)ₘ₄-, and L₃ preferably represents L₁₅-L₂₁-L₁₆-.

L₁₁, L₁₂, L₁₃, L₁₄, L₁₅, and L₁₆ each independently represent -CONH-, -NHCO-, - COO-, -OCO-, -CO-, or -O-.

L₁₁ and L₁₂ are preferably -CONH-, -NHCO-, or -O-. Particularly preferably, L₁₁ is -O-, and L₁₂ is -NHCO-.

L₁₃ and L₁₄ are preferably -NHCO-, -CONH-, or -O-. Particularly preferably, L₁₃ is -CONH-, and L₁₄ is -O-.

L₁₅ is preferably -CONH- or a single bond.

L₁₆ is preferably -NHCO-.

m₁, m₂, m₃, and m₄ each independently represent an integer from 1 to 10, preferably an integer from 2 to 10, more preferably an integer from 2 to 8, and further preferably an integer from 2 to 6.

* represents a binding site with L₄,

L₂₁ preferably represents -(CH₂)₂-(O(CH₂)₂)ₘ₅-.

m₅ represents an integer from 1 to 10, preferably an integer from 1 to 6, and more preferably an integer from 1 to 4.

L₄ is a trivalent linking group, and is preferably 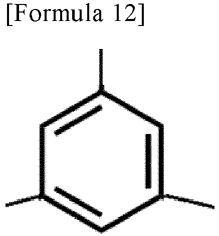 or, 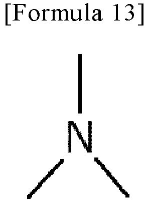 (which is a benzene-derived trivalent linking group or nitrogen atom).

Examples of the halogen represented by Hal may include fluorine (F), chlorine (C1), bromine (Br), iodine (I), astatine (At), tennessine (Ts), and an isotope thereof. Preferred examples of the halogen may include iodine, astatine, and an isotope thereof. Specific examples of the halogen may include ¹²³I, ¹²⁴I, ¹²⁵I, ¹³¹I, ²¹⁰At, and ²¹¹At. Among the above-described halogens, I, ¹²³I, ¹²⁴I, ¹²⁵I, ¹³¹I and ²¹¹At are preferable.

p represents an integer of 1 to 5, preferably an integer of 1 to 3, and particularly preferably 1.

### (2) Method for producing halogenated biotin-modified dimer

As described in the Examples as described later, the halogenated biotin-modified dimer of the present invention can be produced by adding sodium halide (sodium iodide, Na¹²⁵I, etc.) or halogen (²¹¹At) dissolved in methanol to a solution of *N*-Bromo-succinimide or *N*-Iodo-succinimide in a mixture of methanol and acetic acid, then stirring the obtained mixture, and then adding a compound represented by the following formula (A) dissolved in methanol to the reaction mixture, so that the added compound is allowed to react with the reaction mixture. The additive amount of *N*-Bromo-succinimide or *N-*Iodo-succinimide is preferably 0.1 to 1.0 equivalent, more preferably 0.1 to 0.5 equivalents, and further preferably 0.1 to 0.3 equivalents. The additive amount (equivalent) of *N*-Bromo-succinimide or *N*-Iodo-succinimide is preferably set to be larger than the additive amount of sodium halide or halogen. However, if the additive amount of *N*-Bromo-succinimide or *N*-Iodo-succinimide is excessively large, the compound represented by the following formula (A) is likely to be decomposed. Thus, the additive amount of *N*-Bromo-succinimide or *N*-Iodo-succinimide is preferably adjusted to be an appropriate amount. 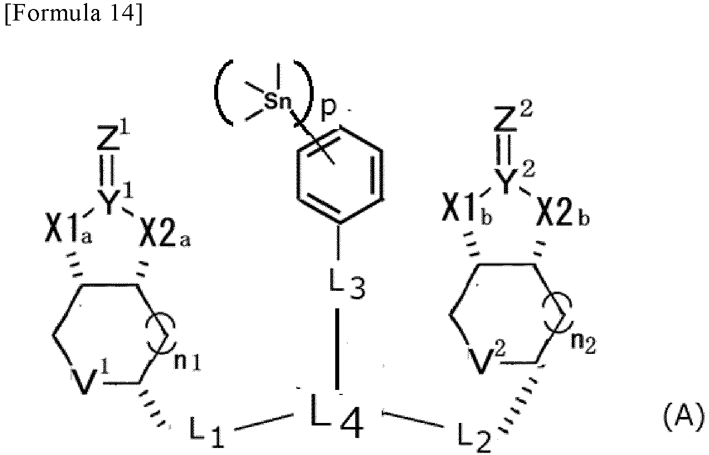 wherein each symbol is as defined in the formula (1).

Alternatively, as described in the Examples later, the halogenated biotin-modified dimer of the present invention can be produced by adding Tetrakis(pyridine)copper(II) triflate (whose additive amount is preferably 1 to 10 equivalents, and more preferably 2 to 8 equivalents) and 3,4,7,8-Tetramethyl-1,10-phenanthroline (whose additive amount is preferably 1 to 10 equivalents, and more preferably 2 to 8 equivalents) and sodium halide to a solution of a compound represented by the following formula (B) in a mixture of methanol and acetonitrile, so that they are allowed to react with one another. 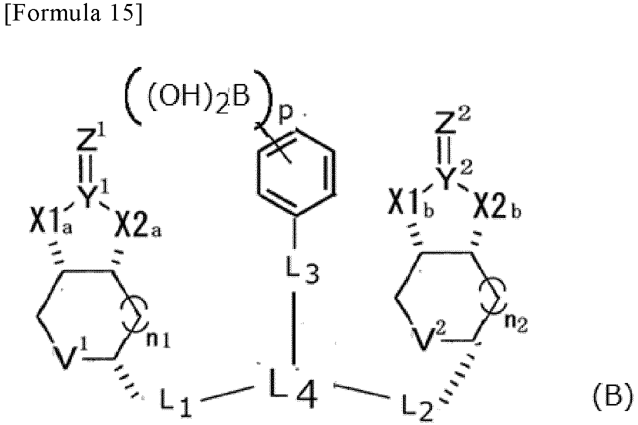 wherein each symbol is as defined in the formula (1).

### (3) Utilization of halogenated biotin-modified dimer

According to the present invention, provided is a therapeutic agent or a diagnostic agent, in which the halogenated biotin-modified dimer of the present invention is combined with a mutant streptavidin-molecular probe conjugate.

As mutant streptavidins used herein, the mutant streptavidins described in International Publication WO2014/129446 and International Publication WO2015/125820 can be used. Particularly preferably, a mutant streptavidin V2122 described in Example 3 of International Publication WO2015/125820 (SEQ ID NO: 4 of International Publication WO2015/125820) can be used.

Examples of the molecular probe used herein may include an antibody, a peptide, a nucleic acid, and an aptamer. Specifically, an antibody, a peptide, a nucleic acid, an aptamer, etc., which target the following antigens specifically expressed in cancer, can be used:

Epiregulin, ROBO 1, 2, 3, and 4, 1-40-β-amyloid, 4-1BB, 5AC, 5T4, ACVR2B, adenocarcinoma antigen, α-fetoprotein, angiopoetin 2, anthrax toxin, AOC3 (VAP-1), B-lymphoma cells, B7-H3, BAFF, β amyloid, C242 antigen, C5, CA-125, carbonic anhydrase 9 (CA-IX), cardiac myosin, CCL11 (eotaxin-1), CCR4, CCR5, CD11, CD18, CD125, CD140a, CD147 (basigin), CD147 (basigin), CD15, CD152, CD154 (CD40L), CD154, CD19, CD2, CD20, CD200, CD22, CD221, CD23 (IgE receptor), CD25 (IL-2 receptor α chain), CD28, CD3, CD30 (TNFRSF8), CD33, CD37, CD38 (cyclic ADP ribose hydrolase), CD4, CD40, CD41 (integrin α-IIb), CD44 v6, CD5, CD51, CD52, CD56, CD6, CD70, CD74, CD79B, CD80, CEA, CFD, ch4D5, CLDN18.2, *Clostridium difficile,* clumping factor A, CSF2, CTLA-4, cytomegalovirus, cytomegalovirus glycoprotein B, DLL4, DR5, *E. coli* Shiga toxin type 1, *E. coli* Shiga toxin type 2, EGFL7, EGFR, endotoxin, EpCAM, episialin, ERBB3, *Escherichia coli,* F protein of respiratory syncytial virus, FAP, fibrin II β chain, fibronectin extra domain-B, folate receptor 1, Frizzled receptor, GD2, GD3 ganglioside, GMCSF receptor α chain, GPNMB, hepatitis B surface antigen, hepatitis B virus, HER1, HER2/neu, HER3, HGF, HIV-1, HLA-DRβ, HNGF, Hsp90, human β amyloid, human scatter factor receptor kinase, human TNF, ICAM-1 (CD54), IFN-α, IFN-γ, IgE, IgE Fc region, IGF-1 receptor, IGF-I, IgG4, IGHE, IL-1β, IL-12, IL-13, IL-17, IL-17A, IL-22, IL-23, IL-4, IL-5, IL-6, IL-6 receptor, IL-9, ILGF2, influenza A hemagglutinin, insulin-like growth factor I receptor, integrin α4, integrin α4β7, integrin α5β1, integrin α7β7, integrin αIIbβ3, integrin αvβ3, integrin γ induced protein, interferon receptor, interferon α/β receptor, ITGA2, ITGB2 (CD18), KIR2D, L-selectin (CD62L), Lewis-Y antigen, LFA-1 (CD11a), lipoteichoic acid, LOXL2, LTA, MCP-1, mesothelin, MS4A1, MUC1, mucin CanAg, myostatin, N-glycolylneuraminic acid, NARP-1, NCA-90 (granulocyte antigen), NGF, NOGO-A, NRP1, Oryctolagus cuniculus, OX-40, oxLDL, PCSK9, PD-1, PDCD1, PDGF-R α, phosphatidylserine, prostate cancer cells, *Pseudomonas aeruginosa,* Rabies virus glycoprotein, RANKL, respiratory syncytial virus, RHD, Rh (Rhesus) factor, RON, RTN4, sclerostin, SDC1, selectin P, SLAMF7, SOST, sphingosine-1-phosphate, TAG-72, TEM1, tenascin C, TFPI, TGFβ1, TGFβ2, TGF-β, TNF-α, TRAIL-R1, TRAIL-R2, tumor antigen CTAA16.88, MUC1 tumor-specific glycosylation, TWEAK receptor, TYRP1 (glycoprotein 75), VEGF-A, VEGFR-1, VEGFR2, vimentin, and VWF.

A fusion body of a molecular probe such as a tumor antigen-specific antibody molecule and a mutant streptavidin is prepared, and the prepared fusion body is then administered to a patient, so that the mutant streptavidin can be accumulated specifically in cancer cells. Subsequently, the halogenated biotin-modified dimer of the present invention having an affinity for the above-described mutant streptavidin is administered to the patient, so that the halogen can be accumulated exactly in the cancer cells. In the present invention, the generation of an antibody is suppressed by a reduction in immunogenicity, and thereby, the clearance of the mutant streptavidin from the body in an early stage caused by the antibody, or shock such as anaphylaxis, can be prevented. Moreover, in the present invention, using the mutant streptavidin of the present invention as an in-vitro diagnostic agent or a clinical diagnostic agent, regarding which the tissue, serum and the like collected from patients are used, noise derived from biotin or a biotin-binding protein present in the tissue, serum and the like can be reduced, so that diagnosis and examination with a higher S/N ratio can be carried out.

Otherwise, in the present invention, a conjugate is prepared by binding a fusion body of a molecular probe such as a tumor antigen-specific antibody molecule and a mutant streptavidin with the halogenated biotin-modified dimer of the present invention, and the thus prepared conjugate can be administered to a patient.

Various types of molecules can be used as antibodies that are to be bound to the mutant streptavidin. Either a polyclonal antibody or a monoclonal antibody may be used. The subclass of the antibody is not particularly limited. Preferably, IgG, and particularly preferably, IgG₁ is used. Furthermore, the term "antibody" includes all of modified antibodies and antibody fragments. Examples of such an antibody include, but are not limited to: a humanized antibody; a human type antibody; a human antibody; antibodies from various types of animals such as a mouse, a rabbit, a rat, a guinea pig and a monkey; a chimeric antibody between a human antibody and an antibody from a different type of animal; diabody; scFv; Fd; Fab; Fab'; and F(ab)'₂.

The conjugate of the mutant streptavidin and the antibody can be obtained by applying a method known to persons skilled in the art. For example, such a conjugate can be obtained by a chemical bond method (US5,608,060). Alternatively, DNA encoding the mutant streptavidin is ligated to DNA encoding an antibody, and using an expression vector or the like, the ligated DNA is then expressed in a host cell, so that such a conjugate can be obtained in the form of a fusion protein. The DNA encoding the mutant streptavidin may be ligated to the DNA encoding an antibody via DNA encoding a suitable peptide, called a linker. The mutant streptavidin-molecular probe conjugate is desirably produced, while keeping the specific binding force between an antibody and a target molecule.

The present invention will be more specifically described in the following examples. However, these examples are not intended to limit the scope of the present invention.

### Examples

### Synthesis of halogenated biotin-modified dimer compound

### General Methods

Nuclear magnetic resonance (NMR) spectra were measured using a JEOL ECX500 (¹H NMR: 500MHz) or JEOL ECS400 (¹H NMR: 400MHz) spectrometer. Chemical shift was expressed in ppm as a value with respect to the residual solvent peak in a deuterated solvent as an internal reference (CDCl₃:δ = 7.26 ppm, CD₃OD:δ = 3.31 ppm). Low-resolution mass spectra (LHMS) were measured using ESI-MS according to Shimadzu LCMS-2020 System. The reaction was traced by thin-layer chromatography (TLC) or low-resolution mass spectrometry (LRMS).

Reverse phase high performance liquid chromatography (HPLC) was carried out using JASCO-HPLC System. Detection was conducted using ultraviolet ray at a wavelength of 254 nm, and a gradient solvent system (acetonitrile/0.1% trifluoroacetic acid MQ solution or 0.1% formic acid MQ solution) was used as a mobile phase. The analysis was carried out using a YMC-Triart-C18 (150 mm x 4.6 mm I.D.) column at a flow rate of 1 mL/min. Fractionation was carried out using a YMC-Triart-C18 (250 mm x 10 mm I.D. or 150 mm x 4.6 mm I.D.) column at a flow rate of 6.3 mL/min in the case of the former column or at a flow rate of 1 mL/min in the case of the latter column.

### < Example 1 >

### (1)

### (3aS,3a'S,4S,4'S,6aR,6a'R)-4,4'-(((((5-((2-(2-(2-(3-(Trimethylstannyl)benzamido)ethoxy)ethoxy)ethyl)carbamoyl)-1,3-phenylene)bis(azanediyl))bis(6-oxohexane-6,1-diyl))bis(azanediyl))bis(5-oxopentane-5,1-diyl))bis(tetrahydro-1H-thieno[3,4-d]imidazol-2(3H)-iminium) formate

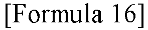

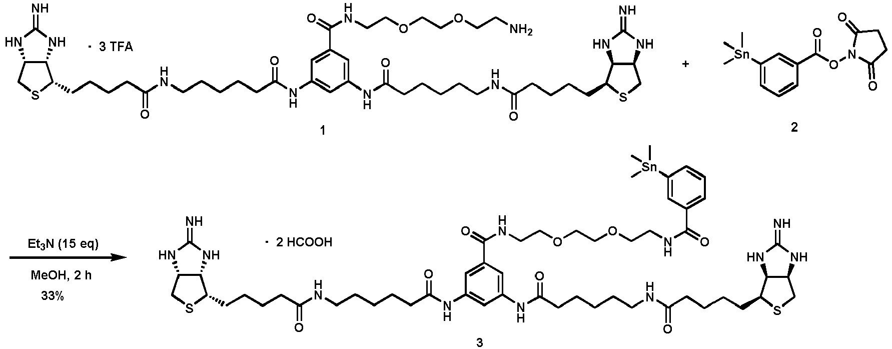

Compound **2** (19.3 mg, 51 µmol) and triethylamine (47.3 µl, 510 µmol) were added to a solution of bisiminobiotin **1** (International Publication WO2015/125820; JP Patent Publication (Kokai) No. 2017-66155 A) (44.7 mg, 34 µmol) in methanol (1 ml), and the obtained mixture was then stirred at room temperature for 2 hours. Thereafter, the solvent was removed under reduced pressure, and the obtained crude product was purified by reverse phase HPLC (gradient: 2% for 2 min; 2-100% for 90 min CH₃CN in 0.1% HCOOH aqueous solution, retention time = 43.6 min, YMC-Triart C18, flow rate = 6.3 ml/min) to obtain the title compound **3** (15.0 mg, a yield of 33%, a white amorphous substance).
¹H NMR (500 MHz, CD₃OD) δ:0.29 (s, 9H), 1.35-1.45 (m, 8H), 1.48-1.80 (m, 16H), 2.18 (t, 4H, *J* =7.4 Hz), 2.37 (t, 4H, *J =* 7.4 Hz), 2.80 (d, 2H, *J =* 13.2 Hz), 2.97 (dd, 2H, *J =* 5.2 Hz, 13.2 Hz), 3.24-3.29 (m, 2H), 3.50-3.58 (m, 4H), 3.62-3.67 (m, 8H), 4.50 (dd, 2H, *J =* 4.6 Hz, 8.0 Hz), 4.70 (dd, 2H, *J =* 4.6 Hz, 8.0 Hz), 7.37 (t, 1H, *J* =7.4 Hz), 7.62 (d, 1H, *J* =6.9 Hz), 7.70 (d, 2H, *J* = 2.1 Hz),7.92-7.94 (m, 1H), 8.01 (t, 1H, *J =* 1.7 Hz), 8.51-8.57 (m, 1H). LRMS (ESI): *m*/*z* 614 [M+2H]²⁺.

### (2)

### (3aS,3a'S,4S,4'S,6aR,6a'R)-4,4'-(((((5-((2-(2-(2-(3-Iodobenzamido)ethoxy)ethoxy)ethyl)carbamoyl)-1,3-phenylene)bis(azanediyl))bis(6-oxohexane-6,1-diyl))bis(azanediyl))bis(5-oxopentane-5,1-diyl))bis(tetrahydro-1H-thieno[3,4-d]imidazol-2(3H)-iminium) 2,2,2-trifluoroacetate

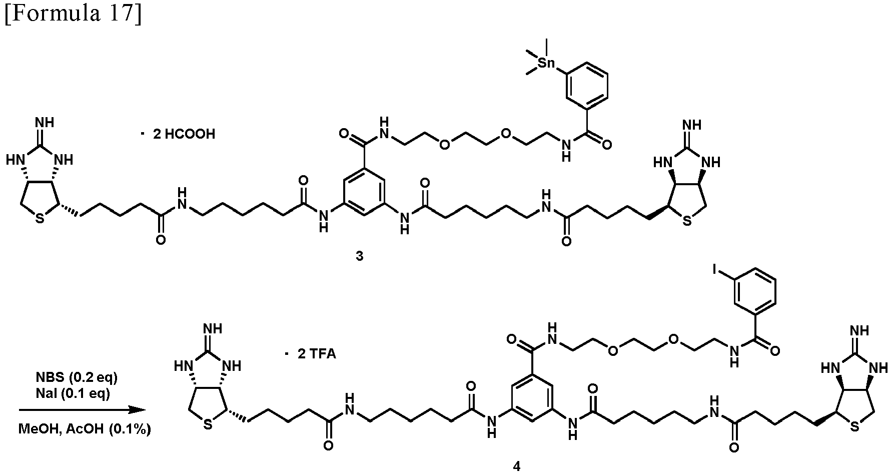

Sodium iodide (0.00114 mg, 0.076 µmol) dissolved in methanol (100 µl) was added to a solution of *N*-Bromo-succinimide (0.0027 mg, 0.152 µmol) in a mixture of methanol (560 µl) and acetic acid (0.76 µl), and the obtained mixture was then stirred at room temperature for 5 minutes. Thereafter, bisiminobiotin **3** (0.1 mg, 0.076 µmol) dissolved in methanol (100 µl) was added to the reaction solution, and the obtained mixture was then stirred at room temperature for 10 minutes. The reaction solution was not concentrated, but was purified by reverse phase HPLC (gradient: 30% for 2 min; 30-100% for 20 min CH₃CN in 0.1% CF₃COOH aqueous solution, retention time = 8.5 min, YMC-Triart C18, flow rate = 1.0 ml/min) to obtain the title compound **4** (a yellow highly viscous oily substance). The obtained product was identical to a preparation synthesized by an alternative method, in terms of the retention time and the measured mass, according to LC-MS analysis.

The title compound 4 is also referred to as "Psyche B-iodine."

¹H NMR (400 MHz, CDCl₃) δ:1.34-1.75 (m, 24H), 2.18 (t, 4H, *J =* 7.2 Hz), 2.38 (t, 4H, *J* = 7.2 Hz), 2.80 (d, 2H*, J* = 13.0 Hz), 2.98 (dd, 2H, *J =* 4.9 Hz, 13.5 Hz), 3.18 (t, 4H, *J =* 7.2 Hz), 3.23-3.28 (m, 2H), 3.55 (t, 4H, *J=* 5.4 Hz), 3.63-3.70 (m, 8H), 4.50 (dd, 2H, *J=* 4.5 Hz, 8.1 Hz), 4.70 (dd, 2H, *J =* 4.5 Hz, 8.1 Hz), 7.19 (t, 1H, *J =* 7.6 Hz), 7.70 (d, 2H, *J* = 1.8 Hz), 7.76-7.80 (m, 1H), 7.83-7.87 (m, 1H), 7.98 (t, 1H, *J* = 1.8 Hz), 8.15 (t, 1H, 1.8 Hz). LRMS (ESI): *m*/*z* 595 [M+2H]²⁺.

### (3)

### (3aS,3a'S,4S,4'S,6aR,6a'R)-4,4'-(((((5-((2-(2-(2-(3-(Iodo-¹²⁵I)benzamido)ethoxy)ethoxy)ethyl)carbamoyl)-1,3-phenylene)bis(azanediyl))bis(6-oxohexane-6,1-diyl))bis(azanediyl))bis(5-oxopentane-5,1-diyl))bis(tetrahydro-1H-thieno[3,4-d]imidazol-2(3H)-iminium) 2,2,2-trifluoroacetate

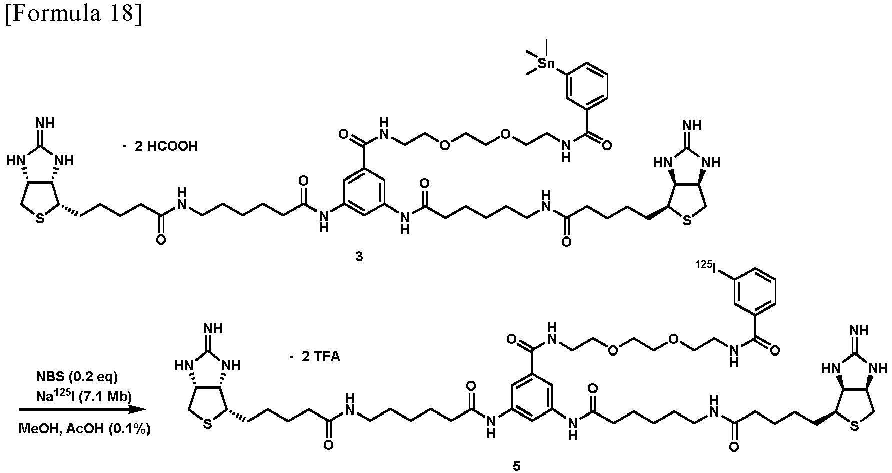

Na¹²⁵**I** (7.1 MBq) dissolved in methanol (100 µl) was added to a solution of *N-*Bromosuccinimide (0.00271 mg, 0.152 µmol) in a mixture of methanol (560 µl) and acetic acid (0.76 µl), and the obtained mixture was then stirred at room temperature for 5 minutes. Thereafter, bisiminobiotin **3** (0.1 mg, 0.076 µmol) dissolved in methanol (100 µl) was added to the reaction solution, and the obtained mixture was then stirred at room temperature for 10 minutes. Thereafter, the reaction solution was not concentrated, but was purified by reverse phase HPLC (30% for 2 min; 30-100% for 20 min CH₃CN in 0.1% CF₃COOH aqueous solution, retention time = 8.5 min, YMC-Triart C18, flow rate = 1.0 ml/min) to obtain the title compound **5.**

### (4)

### (3aS,3a'S,4S,4'S,6aR,6a'R)-4,4'-(((((5-((2-(2-(2-(3-(Astato-²¹¹At)benzamido)ethoxy)ethoxy)ethyl)carbamoyl)-1,3-phenylene)bis(azanediyl))bis(6-oxohexane-6,1-diyl))bis(azanediyl))bis(5-oxopentane-5,1-diyl))bis(tetrahydro-1H-thieno[3,4-d]imidazol-2(3H)-iminium) 2,2,2-trifluoroacetate

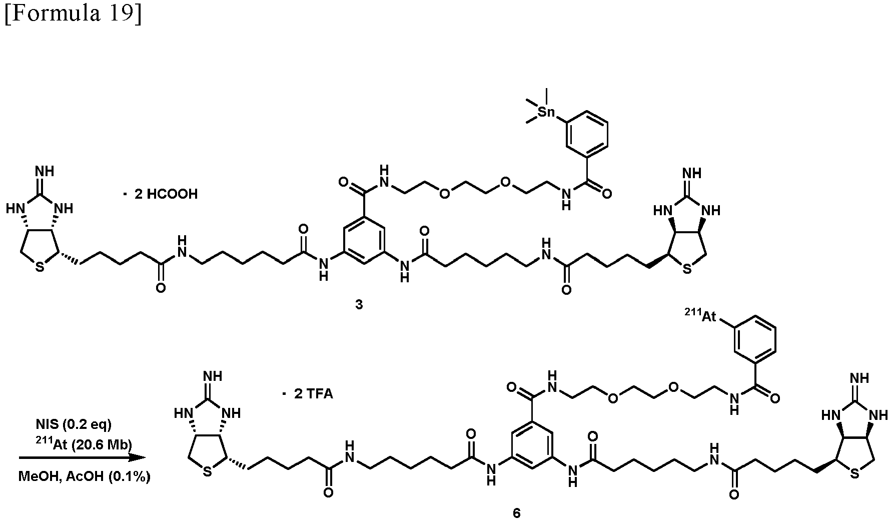

²¹¹At (20.6 MBq) dissolved in methanol (100 µl) was added to a solution of *N-*Iodosuccinimide (0.0034 mg, 0.152 µmol) in a mixture of methanol (560 µl) and acetic acid (0.76 µl), and the obtained mixture was then stirred at room temperature for 1 minute. Thereafter, bisiminobiotin **3** (0.1 mg, 0.076 µmol) dissolved in methanol (100 µl) was added to the reaction solution, and the obtained mixture was then stirred at room temperature for 10 minutes. Thereafter, the reaction solution was not concentrated, but was purified by reserve phase HPLC (30% for 2 min; 30-80% for 13 min CH₃CN in 0.1% CF₃COOH aqueous solution, retention time = 7.0 min, YMC-Triart C18, flow rate = 1.0 ml/min) to obtain the title compound **6.** The title compound 6 is also referred to as "Psyche B-At211."

### < Example 2 >

### (1)

### 2,5-Dioxopyrrolidin-1-yl 3-(4,4,5,5-tetramethyl-1,3,2-dioxaborolan-2-yl)benzoate

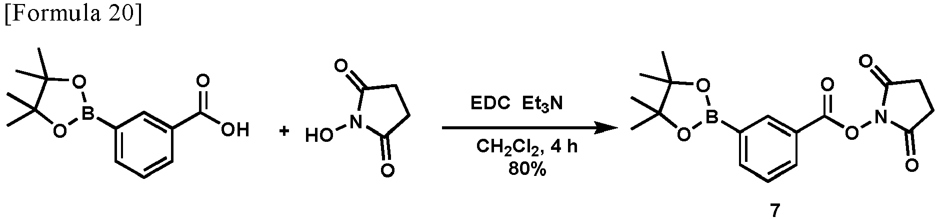

*N*-Hydroxysuccinimide (21.4 mg, 188 µmol) and 1-(3-Dimethylaminopropyl)-3-ethylcarbodiimide Hydrochloride (36 mg, 188 µmol) were added to a solution of 3-(4,4,5,5-Tetramethyl-1,3,2-dioxaborolan-2-yl)benzoic acid in a mixture of dichloromethane (3 ml) and triethylamine (26 µl, 188 µmol), and the obtained mixture was then stirred in an argon atmosphere at room temperature for 4 hours. Thereafter, the solvent was removed under reduced pressure, and water was added to the residue, followed by extraction with ethyl acetate. The organic layer was washed with a saturated ammonium chloride aqueous solution once, then with a saturated sodium hydrogen carbonate aqueous solution once, and then with a saturated saline once. The resultant was dried over sodium sulfate, and the solvent was then removed under reduced pressure to obtain the target compound 7 (42.7 mg, a yield of 80%, a white solid).
¹H NMR (400 MHz, CDCl₃) δ:1.35 (s, 12H), 2.85-2.98 (br, 4H), 7.52 (t, 1H, *J =* 7.6 Hz), 8.06-8.10 (m, 1H), 8.20(dt, 1H, *J =* 1.8 Hz, *J =* 7.6 Hz), 8.59 (s, 1H). LRMS (ESI): *m*/*z* 368 [M+Na]⁺.

### (2)

### (3-((2-(2-(2-(3,5-bis(6-(5-((3aS,4S,6aR)-2-Iminohexahydro-1H-thieno[3,4-d]imidazol-4-yl)pentanamido)hexanamido)benzamido)ethoxy)ethoxy)ethyl)carbamoyl)phenyl)boronic acid

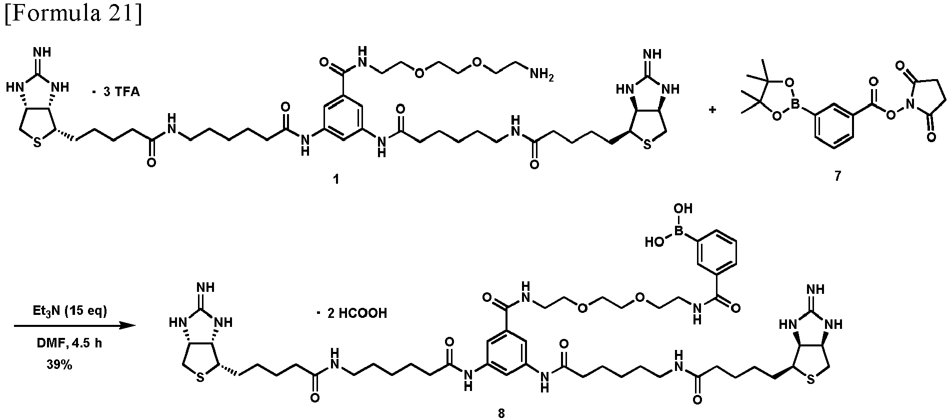

Compound **7** (3.9 mg, 11.5 µmol) and triethylamine (16 µl, 115 µmol) were added to a solution of bisiminobiotin **1** (International Publication WO2015/125820; JP Patent Publication (Kokai) No. 2017-66155 A) (10 mg, 7.68 µmol) in DMF (400 µl), and the obtained mixture was then stirred at room temperature for 4.5 hours. Thereafter, the solvent was removed under reduced pressure, and the obtained crude product was then purified by reverse phase HPLC (gradient: 0% for 5 min; 0-100% for 90 min CH₃CN in 0.1% HCOOH aqueous solution, retention time = 32.5 min, YMC-Triart C18, flow rate = 3.5 ml/min) to obtain the title compound **8** (3.6 mg, a yield of 39%, a white amorphous substance).
¹H NMR (500 MHz, CDCl₃) δ:1.34-1.75 (m, 24H), 2.17 (t, 4H, *J =* 7.4 Hz), 2.37 (t, 4H, *J* = 7.4 Hz), 2.79 (d, 2H, *J =* 13.2 Hz), 2.96 (dd, 2H, *J =* 4.6 Hz, 13.2 Hz), 3.17 (t, 4H, *J =* 6.3 Hz), 3.21-3.28 (m, 2H), 3.50-3.57 (m, 4H), 3.61-3.69 (m, 8H), 4.49 (dd, 2H, *J=* 4.0 Hz, 7.4 Hz), 4.69 (dd, 2H, *J=* 4.6 Hz, 8.0 Hz), 7.35 (t, 1H, *J=* 7.4 Hz), 7.65-7.79 (m, 3H), 7.96-8.03 (m, 2H), 8.50 (s, 1H). LRMS (ESI): *m*/*z* 554 [M+2H]²⁺.

### (3)

### (3aS,3a'S,4S,4'S,6aR,6a'R)-4,4'-(((((5-((2-(2-(2-(3-Iodobenzamido)ethoxy)ethoxy)ethyl)carbamoyl)-1,3-phenylene)bis(azanediyl))bis(6-oxohexane-6,1-diyl))bis(azanediyl))bis(5-oxopentane-5,1-diyl))bis(tetrahydro-1H-thieno[3,4-d]imidazol-2(3H)-iminium) 2,2,2-trifluoroacetate

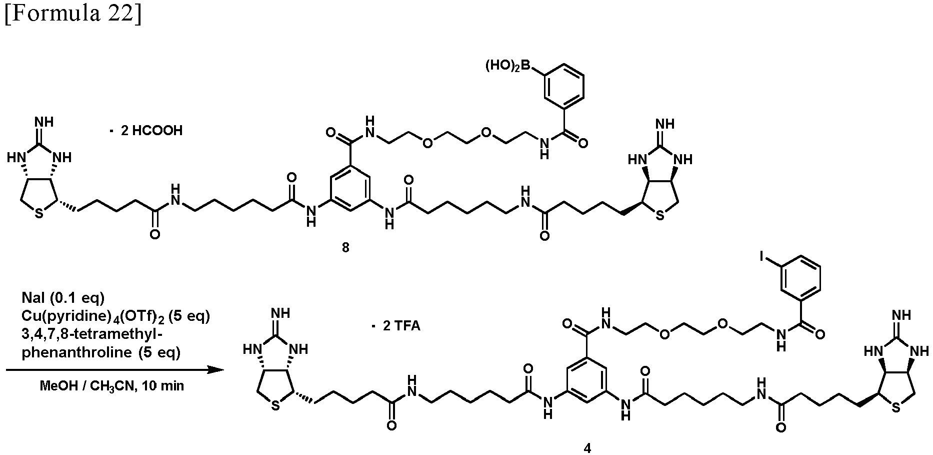

Tetrakis(pyridine)copper(II) triflate (2.8 mg, 4.2 µmol), 3,4,7,8-Tetramethyl-1,10-phenanthroline (0.99 mg, 4.2 µmol) and sodium iodide (0.0125 mg, 0.0834 µmol) were added to a solution of bisiminobiotin **8** in a mixture of methanol (17.5 µl) and acetonitrile (2.5 µl), and the obtained mixture was then stirred at room temperature for 10 minutes. Thereafter, the reaction solution was subjected to LCMS analysis, and as a result, the reaction solution was identical to a preparation synthesized by an alternative method, in terms of retention time and measured mass.

The title compound 4 is also referred to as "Psyche B-iodine."

### < Example 3 >

### Methyl 5-((2S,3S,4R)-3,4-diaminotetrahydrothiophen-2-yl)pentanoate hydrobromide

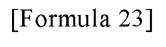

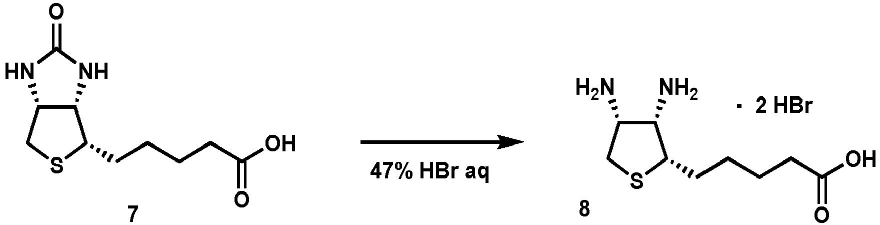

12 ml of a 47% Hydrogen bromide aqueous solution was added to biotin 7 (2.5 g, 10.2 mmol), and the obtained mixture was then stirred under reflux in an argon atmosphere at 150°C for 48 hours. Thereafter, the solvent was removed under reduced pressure. The obtained crude product **8** was used in the subsequent reaction, without being subjected to further purification operations.

### Methyl 5-((2S,3S,4R)-3,4-diaminotetrahydrothiophen-2-yl)pentanoate hydrobromide

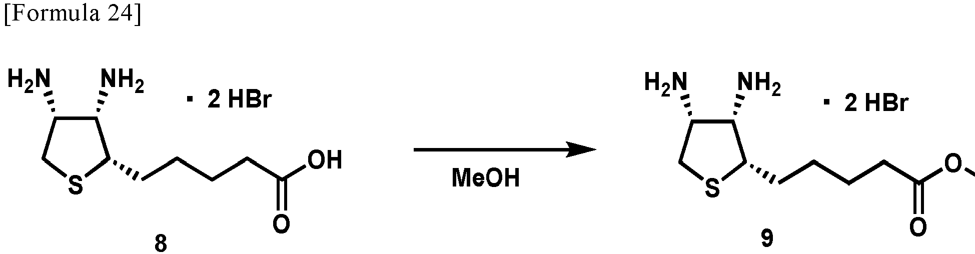

20 ml of Methanol was added to the crude product **8,** and the obtained mixture was then stirred under reflux in an argon atmosphere for 5 hours. Thereafter, the solvent was removed under reduced pressure, and the obtained crude product **9** was used in the subsequent reaction, without being subjected to further purification operations.

### 5-((2S,3S,4R)-3,4-Diaminotetrahydrothiophen-2-yl)pentan-1-ol

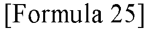

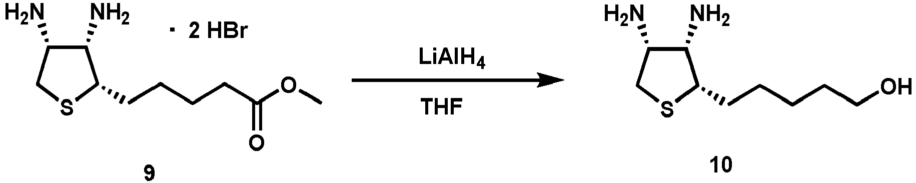

Under cooling on ice, the crude product **9** in a THF (400 ml) solution was added dropwise to a solution of lithium aluminum hydride (1.55 g, 40.9 mmol) in THF (50 ml) over 30 minutes. The obtained mixture was stirred in an argon atmosphere on an ice bath for 3 hours. Thereafter, water (1.5 ml), a 15% NaOH aqueous solution (1.5 ml), and water (4.5 ml) were added to the reaction mixture, and a filtrate obtained by sodium sulfate filtration was then concentrated under reduced pressure. The obtained roughly purified product **10** (1.9 g, a yellow solid) was used in the subsequent reaction, without being subjected to further purification operations.

### Di-tert-butyl ((2S,3S,4R)-2-(5-hydroxypentyl)tetrahydrothiophene-3,4-diyl)dicarbamate

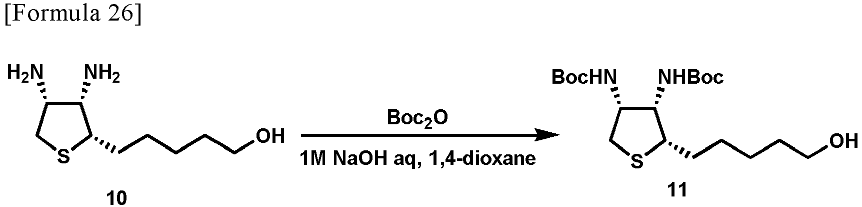

Di-tert-butyl dicarbonate (4.26 g, 20.5 mmol) was added to a solution of the crude product **10** in 1,4-dioxane (20 ml) at room temperature, and then, a 1 M NaOH aqueous solution (20 ml) was added thereto under cooling on ice. The obtained mixture was stirred in an argon atmosphere at room temperature for 15 hours. Thereafter, the solvent was removed under reduced pressure, and a 1 M HCl aqueous solution was then added to the residue, followed by extraction with ethyl acetate. The organic layer was washed with a saturated saline. The resultant was dried over sodium sulfate, and the solvent was then removed under reduced pressure. The obtained roughly purified product was purified by silica gel chromatography (hexane/ethyl acetate = 67 : 33 → 20 : 80 → 0 : 100 → ethyl acetate/methanol = 95 : 5 → 90 : 10) to obtain the target compound **11** (1.77 g, a yield of 46% after 4 stages from the compound **7,** a white amorphous substance).
¹H NMR (500 MHz, CDCl₃) δ:1.20-1.38 (m, 4H), 1.38-1.50 (m, 19H), 1.50-1.57 (m, 2H), 1.57-1.78 (m, 1H), 2.45 (t*, J =* 10.3 Hz, 1H), 3.10-3.27 (m, 1H), 3.45-3.46 (m, 1H), 3.60 (t*, J* = 6.9 Hz, 2H), 4.05-4.25 (m, 1H), 4.25-4.40 (m, 1H), 4.70-4.85 (m, 1H), 4.90-5.05 (m, 1H). LRMS (ESI): *m*/*z* 427 [M+H]⁺.

### Di-tert-butyl ((2S,3S,4R)-2-(5-oxopentyl)tetrahydrothiophene-3,4-diyl)dicarbamate

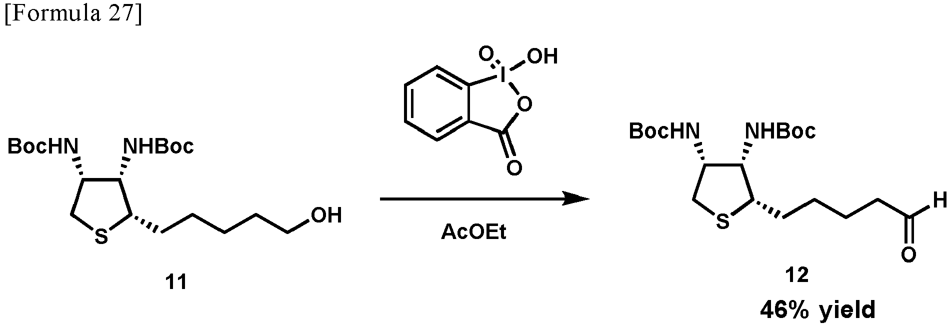

2-Iodoxybenzoic acid (4.08 g, 5.68 mmol, purity: 39%, a commercially available product) was added to a solution of the compound **11** (1.15 g, 2.84 mmol) in ethyl acetate (12ml). The obtained mixture was stirred in an argon atmosphere at 80°C under reflux for 4 hours. Thereafter, the solution was filtrated with Celite, and the filtrate was then removed under reduced pressure. The obtained crude product was purified by silica gel chromatography (hexane/ethyl acetate = 76 : 24 →55 : 45) to obtain the target compound **12** (0.53 g, a yield of 46%, a white amorphous substance).
¹²H NMR (500 MHz, CD₃OD) δ:1.30-1.50 (m, 24H), 2.42 (t, *J =* 5.7 Hz, 2H), 2.47 (t, *J*= 10.3 Hz, 1H), 3.15-3.25 (m, 1H), 3.45-3.55 (m, 1H), 4.10-4.35 (m, 2H), 4.65-4.80 (m, 1H), 4.90-5.00 (m, 1H), 9.75 (t, *J* = 1.7 Hz, 1H) .

### Di-tert-butyl ((2S,3S,4R)-2-(5,5-bis(4-(1,3-dioxoisoindolin-2-yl)butoxy)pentyl)tetrahydrothiophene-3,4-diyl)dicarbamate

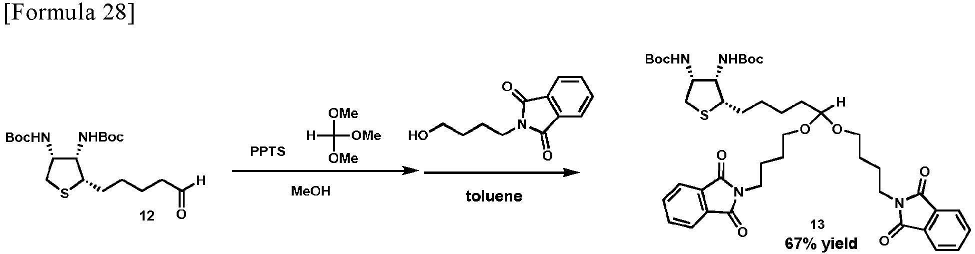

Methyl orthoformate (333 µl, 2.95 mmol) and pyridinium *p*-toluenesulfonate (13.6 mg, 0.059 mmol) were added to a solution of the compound **12** (235.7 mg, 0.59 mmol) in methanol (2 ml) at room temperature. The obtained mixture was stirred in an argon atmosphere at room temperature for 10 hours, and the solvent and the methyl orthoformate were removed under reduced pressure. Toluene (13 ml) and 2-(4-Hydroxybutyl)isoindoline-1,3-dione (1.03 g. 4.68 mmol) were added to the residue, and the thus obtained mixture was then stirred using a Dean-stark device under reflux for 8 hours. Thereafter, the solvent was removed under reduced pressure, and the obtained crude product was purified by silica gel chromatography (hexane/ethyl acetate = 64 : 36 → 46 : 54 → 35 : 65) to obtain the target compound **13** (0.32g, a yield of 67%).
¹H NMR (500 MHz, CDCl₃) δ:1.28-1.80 (m, 34H), 2.46 (t, *J* = 10.3 Hz, 1H), 3.05-3.35 (m, 1H), 3.34-3.44 (m, 2H), 3.47-3.62 (m, 3H), 3.70, (t, *J=* 7.4 Hz, 4H), 4.18-4.23 (m, 1H), 4.28-4.33 (m, 1H), 4.39 (t, *J=* 5.2 Hz, 1H), 4.74-4.81 (m, 1H), 4.97-5.03 (m, 1H), 7.68-7.74 (m, 4H), 7.80-7.84 (m, 4H).

### 2-(4-((5-((2S,3S,4R)-3,4-Diaminotetrahydrothiophen-2-yl)pentyl)oxy)butyl)isoindoline-1,3-dione

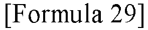

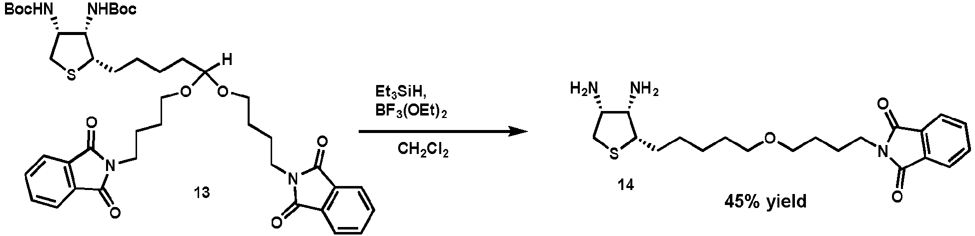

Triethylsilane (468 µl, 2.93 mmol) and boron trifluoride diethyl etherate (234 µl, 0.881 mmol) were added to the compound **13** (302 mg, 0.367 mmol) in a dichloromethane (7.2 ml) solution at room temperature, and the obtained mixture was then stirred in an argon atmosphere at room temperature for 11 hours. Thereafter, methanol (500 µl) was added to the reaction solution, and the obtained mixture was then stirred at room temperature for 1 hour. After that, the solvent was removed under reduced pressure. The obtained crude product was purified by silica gel chromatography (chloroform/methanol = 99 : 1 → 75 : 25 → 65 : 35). A saturated sodium bicarbonate solution was added to the obtained compound, and the obtained mixture was then extracted with dichloromethane. The extract was dried over sodium sulfate, and the solvent was then removed under reduced pressure to obtain the target compound **14** (67.6 mg, a yield of 45%, a yellow oily substance).
¹H NMR (400 MHz, CDCl₃) δ:1.32-1.62 (m, 9H), 1.67-1.76 (m, 3H), 2.55 (d*, J =* 13.2 Hz, 1H), 2.93 (dd, *J=* 5.7 Hz, 12.6 Hz, 1H), 3.20-3.30 (m, 1H), 3.41 (t, *J=* 6.3 Hz, 2H), 3.45 (t*, J =* 6.3 Hz, 2H), 3.68 (t, *J =* 6.9 Hz, 2H) 3.82 (t, *J =* 5.7 Hz, 1H), 3.94 (t, *J =* 6.3 Hz, 1H), 7.76-7.81 (m, 2H), 7.82-7.87 (m, 2H). LRMS (ESI): *m*/*z* 406 [M+H]⁺.

### tert-Butyl ((3aS,4S,6aR,E)-4-(5-(4-(1,3-dioxoisoindolin-2-yl)butoxy)pentyl)tetrahydro-1H-thieno[3,4-d]imidazol-2(3H)-ylidene)carbamate

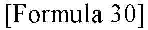

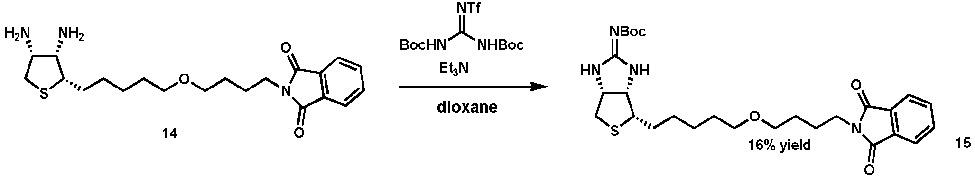

A Goodman reagent (42 mg, 0.107mmol) dissolved in dioxane (400 µl) was added to a solution of the compound **14** (43.5 mg, 0.107 mmol) in a mixture of dioxane (300 µl) and triethylamine (30 µl, 0.214 mmol) at room temperature. The obtained mixture was stirred in an argon atmosphere at room temperature for 21 hours, and the solvent was then removed under reduced pressure. The obtained crude product was purified by silica gel chromatography (chloroform/methanol = 100 : 0 → 10:1 → 5 : 1) to obtain the target compound **15** (9.1 mg, a yield of 16%).
¹H NMR (500 MHz, CDCl₃) δ:1.32-1.50 (m, 4H), 1.43 (s, 9H), 1.51-1.69 (m, 5H), 1.71-1.81 (m, 3H), 2.86-2.95 (m, 2H), 3.18-3.24 (m, 1H), 3.38 (t, *J=* 6.3 Hz, 2H), 3.42 (t, *J=* 6.3 Hz, 2H), 3.71 (t, *J =* 6.9 Hz, 2H), 4.42 (dd, *J =* 5.2 Hz, 8.0 Hz, 1H),4.62 (m, 1H), 7.67-7.73 (m, 2H), 7.80-7.86 (m, 2H). LRMS (ESI): *m*/*z* 531 [M+H]⁺.

### tert-Butyl ((3aS,4S,6aR,E)-4-(5-(4-aminobutoxy)pentyl)tetrahydro-1H-thieno[3,4-d]imidazol-2(3H)-ylidene)carbamate

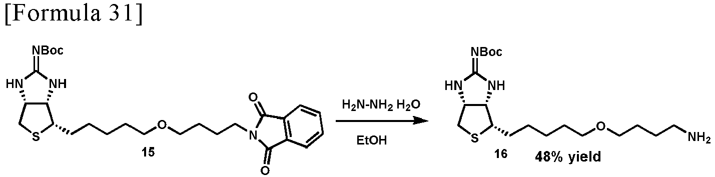

Hydrazine hydrate (10 mg, 0.3 mmol) was added to a solution of the compound **15** (31.7 mg, 0.06 mmol) in ethanol (500 µl) at room temperature. The reaction solution was stirred at 50°C for 10 hours. Thereafter, the reaction solution was subjected to suction filtration, and a filtrate was then removed under reduced pressure. The obtained crude product was purified by silica gel chromatography (amino silica, chloroform/methanol = 100 : 0 → 94 : 6) to obtain the target compound **16** (11.8 mg, a yield of 49%).
¹H NMR (400 MHz, CDCl₃) δ:1.40-1.50 (m, 13H), 1.51-1.69 (m, 7H), 1.72-1.82 (m, 1H), 2.69 (t, *J=* 6.7 Hz, 2H), 2.81 (d, *J=* 6.3 Hz, 1H), 2.97 (dd, *J* = 4.9 Hz, 13.0 Hz, 1H), 3.24-3.32 (m, 1H), 3.40-3.48 (m, 4H), 4.41 (dd, *J=* 4.5 Hz, 8.1 Hz, 1H), 4.62 (dd, *J=* 4.5 Hz, 8.1 Hz, 1H). LRMS (ESI): *m*/*z* 401 [M+H]⁺.

### 14-(Carboxymethyl)-2,2-dimethyl-4-oxo-3,8,11-trioxa-5,14-diazahexadecan-16-oic acid

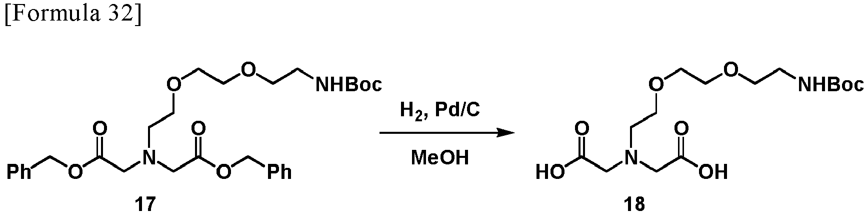

10% Palladium carbon (24.1 mg, 22.6 µmol) was added to a solution of the compound 17 (AMGEN INC.WO2006/14645, 2006, A1) (131.1 mg, 240.7 mmol) in methanol (5 ml), and the obtained mixture was then stirred in a hydrogen atmosphere at room temperature for 3 hours. Thereafter, the reaction solution was filtrated with Celite, and the solvent was then removed under reduced pressure. The obtained crude product **18** (93.3 mg, yield: quant., a colorless highly-viscous oily substance) was used in the subsequent reaction, without being subjected to further purification operations.

### tert-Butyl (22-((3aS,4S,6aR,E)-2-((Tert-butoxycarbonyl)imino)hexahydro-1H-thieno[3,4-d]imidazol-4-yl)-9-(2-((4-((5-((3aS,4S,6aR,Z)-2-((tert-butoxycarbonyl)imino)hexahydro-1H-thieno[3,4-d]imidazol-4-yl)pentyl)oxy)butyl)amino)-2-oxoethyl)-11 -oxo-3,6,17-trioxa-9,12-diazadocosyl)carbamate

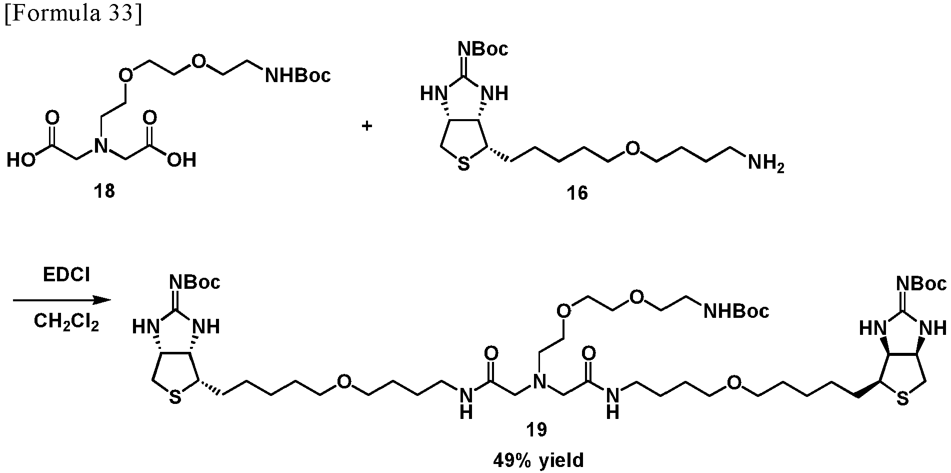

The Compound **16** (12.9 mg, 32.2 µmol) and 1-ethyl-3-(3-dimethylaminopropyl) carbodiimide hydrochloride (6.9 mg, 36 (µmol) were added to a solution of the compound **18** (6.4 mg, 17.5 µmol) in dichloromethane (400 µl) at room temperature. The obtained mixture was stirred in an argon atmosphere at room temperature for 36 hours, and the solvent was then removed under reduced pressure. The obtained crude product was purified by silica gel chromatography (amino silica, chloroform/methanol = 200 : 1 → 50 : 1 → 30 : 1→ 20 : 1 → 10:1 → 5 : 1 → 3 : 1) to obtain the target compound **19** (9.6 mg, a yield of 49%).
¹H NMR (400 MHz, CD₃OD) δ:1.34-1.50 (m, 35H), 1.51-1.64 (m, 14H), 1.72-1.82 (m, 2H), 2.81 (d, *J =* 12.6 Hz, 2H), 2.97 (dd, *J =* 4.9 Hz, 12.6 Hz, 2H), 3.18-3.30 (m, 10H), 3.39-3.65 (m, 20H), 4.41 (dd, *J=* 4.5 Hz, 8.1 Hz, 2H), 4.63 (dd, *J=* 4.5 Hz, 8.1 Hz, 2H). LRMS (ESI): *m*/*z* 565 [M+2H]²⁺.

### 2,2'-((2-(2-(2-Aminoethoxy)ethoxy)ethyl)azanediyl)bis(N-(4-((5-((3aS,4S,6aR)-2-iminohexahydro-1H-thieno[3,4-d]imidazol-4-yl)pentyl)oxy)butyl)acetamide) 2,2,2-trifluoroacetate

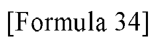

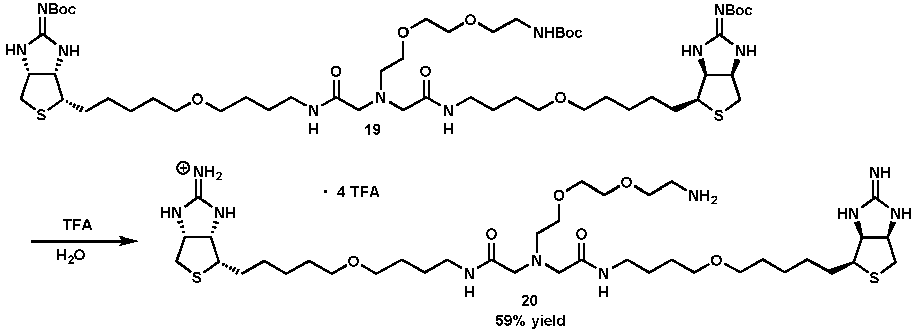

Trifluoroacetic acid (400 µl) was added to an aqueous solution (200 µl) of the compound **19** (5.2 mg, 4.6 µmol) at room temperature. The obtained mixture was stirred in an argon atmosphere at room temperature for 18 hours, and the solvent was then removed under reduced pressure. The obtained crude product was purified by reverse phase HPLC (gradient: 0% for 5 min; 0-100% for 90 min CH₃CN in 0.1% CF₃COOH aqueous solution, YMC-Triart C18, flow rate = 3.5 ml/min) to obtain the target compound **20** (3.5 mg, a yield of 59%).
¹H NMR (500 MHz, CD₃OD) δ:1.34-1.50 (m, 8H), 1.52-1.63 (m, 14H), 1.71-1.81 (m, 2H), 2.82 (d, *J =* 13.2 Hz, 2H), 2.99 (dd, *J =* 4.6 Hz, 13.2 Hz, 2H), 3.14 (t, *J =* 4.6 Hz, 4H), 3.20-3.30 (m, 6H), 3.43 (t, *J=* 6.3 Hz, 8H), 3.60-3.74 (m, 12H), 4.53 (dd, *J=* 4.0 Hz, 8.0 Hz, 2H), 4.72 (dd, *J=* 4.6 Hz, 8.0 Hz, 2H). LRMS (ESI): *m*/*z* 415 [M+2H]²⁺.

### 2-((4-((5-((3aS,4S,6aR)-2-Iminiohexahydro-1H-thieno[3,4-d]imidazol-4-yl)pentyl)oxy)butyl)amino)-N-(2-((4-((5-((3aS,4S,6aR)-2-iminiohexahydro-1H-thieno[3,4-d]imidazol-4-yl)pentyl)oxy)butyl)amino)-2-oxoethyl)-2-oxo-N-(2-(2-(2-(3-(trimethylstannyl)benzamido)ethoxy)ethoxy)ethyl)ethan-1-aminium formate

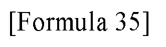

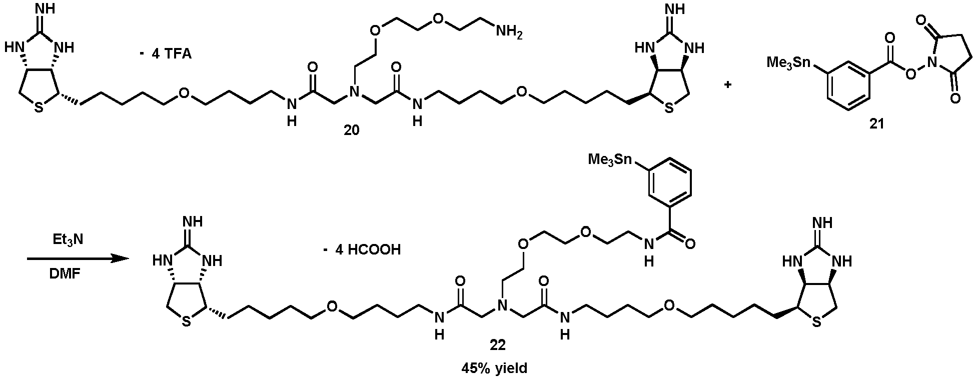

Compound **21** (1.1 mg, 2.99 µmol) and triethylamine (5.7 µl, 40.8 µmol) were added to a solution of the compound **20** (3.5 mg, 2.72 µmol) in DMF (300 µl) at room temperature. The obtained mixture was stirred in an argon atmosphere at room temperature for 3 hours, and the solvent was then removed under reduced pressure. The obtained crude product was purified by reverse phase HPLC (gradient: 0% for 5 min; 2-100% for 90 min CH₃CN in 0.1% HCOOH aqueous solution, retention time: 42.0 min, YMC-Triart C18, flow rate = 3.5 ml/min) to obtain the target compound **22** (1.5 mg, a yield of 45%). LRMS (ESI): *m*/*z* 366 [M+3H]³⁺.

### 2,2'-((2-(2-(2-(3-Iodobenzamido)ethoxy)ethoxy)ethyl)azanediyl)bis(N-(4-((5-((3aS,4S,6aR)-2-iminohexahydro-1H-thieno[3,4-d]imidazol-4-yl)pentyl)oxy)butyl)acetamide) 2,2,2-trifluoroacetate

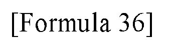

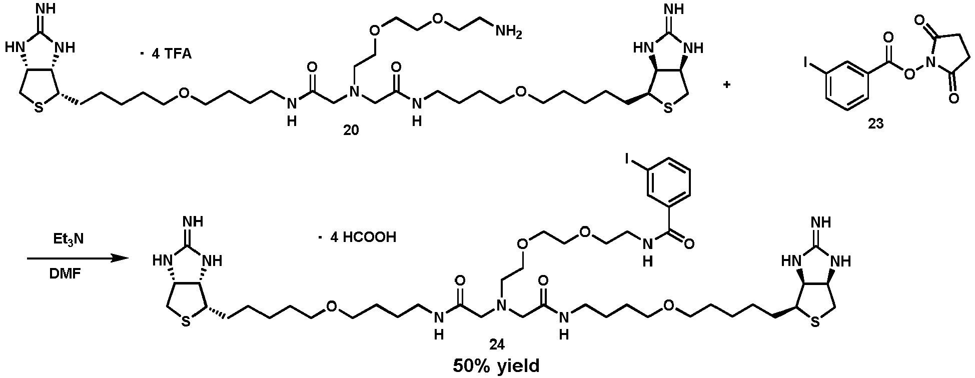

Compound **23** (0.7 mg, 2.06 µmol) dissolved in DMF (100 µl) and triethylamine (4 µl, 28.3 µmol) were added to a solution of the bisiminobiotin **20** (2.4 mg, 1.87 µmol) in DMF (150 µl) at room temperature. The obtained mixture was stirred in an argon atmosphere at room temperature for 2 hours, and the solvent was then removed under reduced pressure. The obtained crude product was purified by reverse phase HPLC (gradient: 0% for 5 min; 2-100% for 90 min CH₃CN in 0.1% CF₃COOH aqueous solution, retention time: 47.6 min, YMC-Triart C18, flow rate = 3.5 ml/min) to obtain the target compound **24** (1.3 mg, a yield of 50%). LRMS (ESI): *m*/*z* 354 [M+3H]³⁺.

The target compound **24** is also referred to as "Psyche P-iodine."

### 2,2'-((2-(2-(2-(3-Iodobenzamido)ethoxy)ethoxy)ethyl)azanediyl)bis(N-(4-((5-((3aS,4S,6aR)-2-iminohexahydro-1H-thieno[3,4-d]imidazol-4-yl)pentyl)oxy)butyl)acetamide) 2,2,2- trifluoroacetate

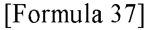

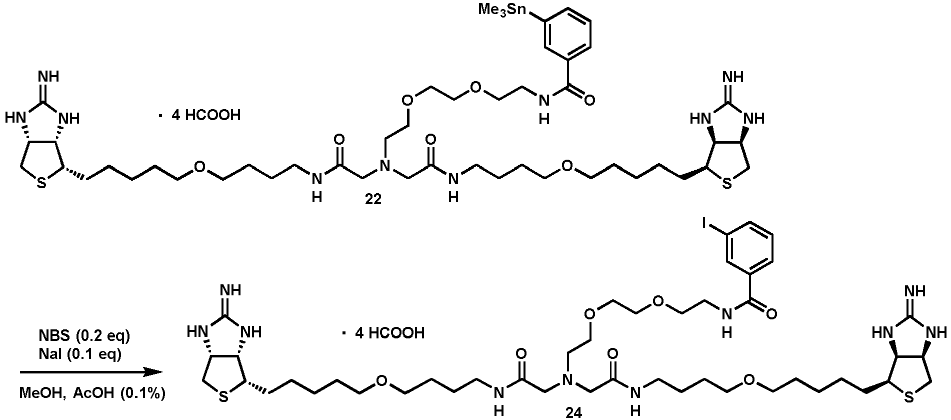

Sodium iodide (0.00121 mg, 0.081 µmol) dissolved in methanol (100 µl) was added to a solution of *N*-Bromo-succinimide (0.00288 mg, 0.162 µmol) in a mixture of methanol (610 µl) and acetic acid (0.81 µl), and the obtained mixture was then stirred at room temperature for 5 minutes. Thereafter, the bisiminobiotin **22** (0.1 mg, 0.081 µmol) dissolved in methanol (100 µl) was added to the reaction solution, and the thus obtained mixture was then stirred at room temperature for 10 minutes. Thereafter, the reaction solution was subjected to LC-MS analysis, and as a result, the reaction solution was identical to the preparation (**24**) synthesized by the above-described method, in terms of the retention time and the measured mass. The target compound **24** is also referred to as "Psyche P-iodine."

### < Production of CEA-V2122 >

V2122 is a mutant streptavidin described in Example 3 of International Publication WO2015/125820 (SEQ ID NO: 4 shown in International Publication WO2015/125820). The amino acid sequence of V2122 (a sequence having 6xHis tag at the C-terminus) is as set forth in SEQ ID NO: 1 in the sequence listing.

scFv-V2122 is prepared by binding a single-chain antibody (scFv) against CEACAM5 with the above-described V2122. This scFv-type anti-CEACAM5 antibody is an scFv sequence described in a patent document US7626011B2. The amino acid sequence of the scFv-type anti-CEACAM5 antibody is as set forth in SEQ ID NO: 2 in the sequence listing. In addition, the amino acid sequence of CEA-V2122 prepared by binding the scFv-type anti-CEACAM5 antibody with V2122 via an amino acid linker (GGGGSGGGG) (SEQ ID NO: 11) is as set forth in SEQ ID NO: 3 in the sequence listing.

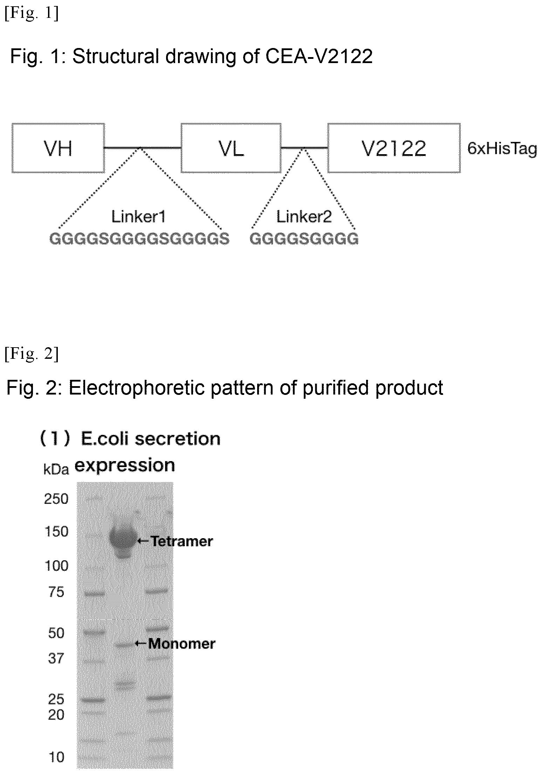

For the expression of a CEA-V2122 fusion protein, the DNA codon of a CEA-V2122 gene sequence, in which a pelB signal for secretion and expression in *Escherichia coli* had been incorporated into the N-terminus and a 6xHis-Tag sequence had been incorporated into the C-terminus, was optimized for *Escherichia coli,* thereby synthesizing an artificial gene. This amino acid sequence is as set forth in SEQ ID NO: 4 in the sequence listing, and the DNA sequence is as set forth in SEQ ID NO: 5 in the sequence listing. Moreover, an outline of a domain structure is shown in Fig. 1.

As a specific protein expression vector, a vector prepared by incorporating a chaperone skp gene into MCS2 of a pETDuet1 vector was used. Regarding the skp gene, the DNA codon was optimized for *Escherichia coli* based on the amino acid sequence as set forth in SEQ ID NO: 6 in the sequence listing, thereby synthesizing an artificial gene. The synthesized skp gene was amplified by PCR, using the primers (AAGGAGATATACATATGGATAAAATTGCCATTGTTAATAT (SEQ ID NO: 12) and TTGAGATCTGCCATATGTTATTTCACTTGTTTCAGAACG (SEQ ID NO: 13)), and the amplified gene was then cloned into MCS2 of the pETDue1 vector linearized with the restriction enzyme NdeI, using In-Fusion HD Cloning Kit, so as to obtain pETDuet_skp. Subsequently, the CEA-V2122 gene was incorporated into MCS1 of pETDuet_skp. Specifically, the artificially synthesized CEA-V2122 gene was amplified by PCR, using the primers (AGAAGGAGATATACCATGAAATATCTGCTGCCGAC (SEQ ID NO: 14) and CGCCGAGCTCGAATTTTAATGATGGTGATGATGATG (SEQ ID NO: 15)). Moreover, pETDuet_skp was linearized by PCR, using the primers (GGTATATCTCCTTCTTAAAGTTAAAC (SEQ ID NO: 16) and AATTCGAGCTCGGCGCGCCTGCAG (SEQ ID NO: 17)). The CEA-V2122 amplified by PCR and the linearized pETDuet_skp were subjected to cloning using In-Fusion HD Cloning Kit. The cloned vector was confirmed by sequencing, in terms of a gene sequence incorporated therein, and it was referred to as pETDuet_CEA-V2122_skp.

For the expression of the protein, pETDuet_CEA-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium to 100-fold dilution, and culture was then carried out at 37°C until OD (600nm) = 0.5 to 2.0. Subsequently, IPTG was added to the culture to a final concentration of 0.5 mM, and the obtained mixture was then cultured at 37°C for 4 hours. Thereafter, a culture supernatant was recovered and was then preserved at 4°C.

The CEA-V2122 protein was roughly purified according to a batch method utilizing 6xHis-Tag added to the C-terminus. Specifically, cOmplete His-Tag Purification Resin equilibrated with buffer A(50 mM Tris-HCl, 0.2 M NaCl, 1 mM EDTA, and 5 mM Imidazole; pH 8.0) was added to the culture supernatant preserved at 4°C. The obtained mixture was stirred from 2 hours to overnight at 4° C, so that the protein was allowed to bind to the resin. Subsequently, the resin was recovered into a column, and a 20 column volume of washing operation was performed with buffer A. Thereafter, a roughly purified product of CEA-V2122 was recovered by elution with buffer B (50 mM Tris-HCl, 0.2 M NaCl, 1 mM EDTA, and 400 mM Imidazole; pH 8.0).

Subsequently, the roughly purified product was purified using a Protein L column. Specifically, 1 mL of Capto L (GE Healthcare Life Sciences) was filled into a PD-10 column and was then equilibrated with 10 column volume of PBS, and the aforementioned roughly purified product was then applied thereto. Thereafter, the resultant was washed with 10 column volume of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare Life Science), the buffer was replaced with PBS, and centrifugal concentration was further carried out using Vivaspin Turbo 4 (MWCO 100,000) to obtain a finally purified product. After completion of SDS-PAGE electrophoresis, the purity of tetramer CEA-V2122 was assayed by CBB staining. The results are shown in Fig. 2. As SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 2, it was confirmed that the purified CEA-V2122 comprises an approximately 150-kDa tetramer as a main component.

### < Production of Herceptin-V2122 >

V2122 is a mutant streptavidin described in Example 3 of International Publication WO2015/125820 (SEQ ID NO: 4 shown in International Publication WO2015/125820). The amino acid sequence of V2122 is as set forth in SEQ ID NO: 1 in the sequence listing.

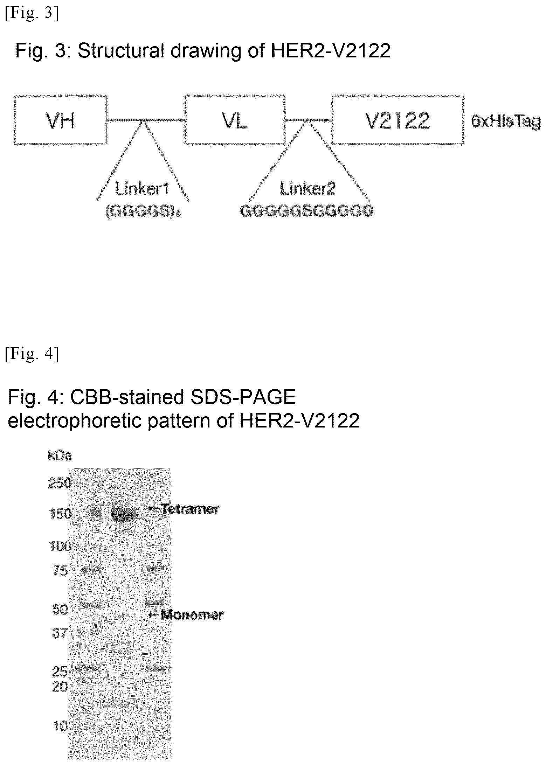

scFv-V2122 is prepared by binding a single-chain antibody (scFv) against HER2 (ERBB2) with the above-described V2122. This scFv-type anti-HER2 antibody is an scFv sequence described in Zhang H, et al., Therapeutic potential of an anti-HER2 single chain antibody-DM1 conjugates for the treatment of HER2-positive cancer. Signal Transduct Target Ther. 2017 May 19; 2: 17015. doi: 10.1038/sigtrans. 2017.15. The amino acid sequence of the scFv-type anti-HER2 antibody is as set forth in SEQ ID NO: 7 in the sequence listing. In addition, the structural drawing of HER2-V2122 prepared by binding the scFv-type anti-HER2 antibody with V2122 via an amino acid linker (GGGGGSGGGGG) (SEQ ID NO: 18) is shown in Fig. 3, and the amino acid sequence of HER2-V2122 is as set forth in SEQ ID NO: 8 in the sequence listing.

For the expression of a HER2-V2122 fusion protein, the DNA codon of a HER2-V2122 gene sequence, in which a pelB signal for secretion and expression in *Escherichia coli* had been incorporated into the N-terminus and a 6xHis-Tag sequence had been incorporated into the C-terminus, was optimized for *Escherichia coli,* thereby synthesizing an artificial gene. This amino acid sequence is as set forth in SEQ ID NO: 9 in the sequence listing, and the DNA sequence is as set forth in SEQ ID NO: 10 in the sequence listing.

As a specific protein expression vector, a vector prepared by incorporating a chaperone skp gene into MCS2 of a pETDuet1 vector was used. Regarding the skp gene, the DNA codon was optimized for *Escherichia coli* based on the amino acid sequence as set forth in SEQ ID NO: 6 in the sequence listing, thereby synthesizing an artificial gene. The synthesized skp gene was amplified by PCR, using the primers (AAGGAGATATACATATGGATAAAATTGCCATTGTTAATAT (SEQ ID NO: 12) and TTGAGATCTGCCATATGTTATTTCACTTGTTTCAGAACG (SEQ ID NO: 13)), and the amplified gene was then cloned into MCS2 of the pETDue1 vector linearized with the restriction enzyme NdeI, using In-Fusion HD Cloning Kit, so as to obtain pETDuet_skp. Subsequently, the HER2-V2122 gene was incorporated into MCS1 of pETDuet_skp. Specifically, the artificially synthesized HER2-V2122 gene was amplified by PCR, using the primers (AGAAGGAGATATACCATGAAATATCTGCTGCCGAC (SEQ ID NO: 14) and CGCCGAGCTCGAATTTTAATGATGGTGATGATGATG (SEQ ID NO: 15)). Moreover, pETDuet_skp was linearized by PCR, using the primers (GGTATATCTCCTTCTTAAAGTTAAAC (SEQ ID NO: 16) and AATTCGAGCTCGGCGCGCCTGCAG (SEQ ID NO: 17)). The CEA-V2122 amplified by PCR and the linearized pETDuet_skp were subjected to cloning using In-Fusion HD Cloning Kit. The cloned vector was confirmed by sequencing, in terms of a gene sequence incorporated therein, and it was referred to as pETDuet_HER2-V2122_skp.

For the expression of the protein, pETDuet_HER2-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium to 100-fold dilution, and culture was then carried out at 37°C until OD (600nm) = 0.5 to 2.0. Subsequently, IPTG was added to the culture to a final concentration of 0.5 mM, and the obtained mixture was then cultured at 37°C for 4 hours. Thereafter, a culture supernatant was recovered and was then preserved at 4°C.

The HER2-V2122 protein was roughly purified according to a batch method utilizing 6xHis-Tag added to the C-terminus. Specifically, cOmplete His-Tag Purification Resin equilibrated with buffer A(50 mM Tris-HCl, 0.2 M NaCl, 1 mM EDTA, and 5 mM Imidazole; pH 8.0) was added to the culture supernatant preserved at 4°C. The obtained mixture was stirred from 2 hours to overnight at 4° C, so that the protein was allowed to bind to the resin. Subsequently, the resin was recovered into a column, and a 20 column volume of washing operation was performed with buffer A. Thereafter, a roughly purified product of HER2-V2122 was recovered by elution with buffer B (50 mM Tris-HCl, 0.2 M NaCl, 1 mM EDTA, and 400 mM Imidazole; pH 8.0).

Subsequently, the roughly purified product was purified using a Protein L column. Specifically, 1 mL of Capto L (GE Healthcare) was filled into a PD-10 column and was then equilibrated with 10 column volume of PBS, and the aforementioned roughly purified product was then applied thereto. Thereafter, the resultant was washed with 10 column volume of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare), the buffer was replaced with PBS, and centrifugal concentration was further carried out using Vivaspin Turbo 4 (MWCO 100,000) to obtain a finally purified product. The purified product was subjected to CBB staining, and the purity of tetramer HER2-V2122 was assayed. The results are shown in Fig. 4. As SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 4, it was confirmed that the purified HER2-V2122 comprises an approximately 150-kDa tetramer as a main component.

### Test Example 1: Analysis of affinity of CEA-V2122 for Psyche B-iodine and for Psyche P-iodine

The data regarding the affinity of CEA-V2122 for Psyche B-iodine and for Psyche P-iodine were obtained in accordance with the description of Japanese Patent Application 2018-247363. Briefly stating, using an amine coupling kit (GE Healthcare Life Sciences), Sensor Chip CM5 (GE Healthcare Life Science) was set to be a target value that was 5000 RU, and the purified CEA-V2122 was immobilized. With regard to the concentration of the analyte, 5 types of two-fold serial dilutions from 1E-08 M to 6.25E-10 M were used. Biacore T200 (GE Healthcare Life Sciences) was used, the measurement temperature was set to be 25°C, and kinetics data were then obtained according to Single-Cycle Kinetics analysis. Using Biacore T200 Evaluation Software, version 2.0 (GE Healthcare Life Sciences), the obtained data were subjected to curve fitting in a Bivalent Analyze mode, and the evaluation value of each parameter was obtained. The dissociation constant K_{D} is obtained according to Kd/Ka = K_{D}. The results are shown in Table 1. From the results, it was confirmed that Psyche B-iodine and Psyche P-iodine form a stable bond with CEA-V2122.

**[Table 1]**

| | Ka (Ms⁻¹) | Kd (s⁻¹) | K_{D} (M) |
|---|---|---|---|
| Psyche B-iodine | 2.9E+04 | 6.4E-07 | 2.2E-11 |
| Psyche P-iodine | 6.8E+03 | 3.1E-05 | 4.5E-09 |

### Test Example 2: Confirmation of binding conjugate of astatine-labeled Psyche B and Herceptin-Cupid by using magnetic beads

In order to confirm whether or not astatine-labeled Psyche B has binding ability to Cupid, a binding assay was carried out using Herceptin-Cupid and Ni-NTA magnetic beads (manufactured by Thermo) that bind to a 6xHis-tag added to Herceptin-Cupid. Psyche B-At211 used herein was prepared according to Example 1(4), whereas Herceptin-Cupid was prepared according to the method described in Japanese Patent Application 2018-247363, as described above. The specific assay method is as follows.

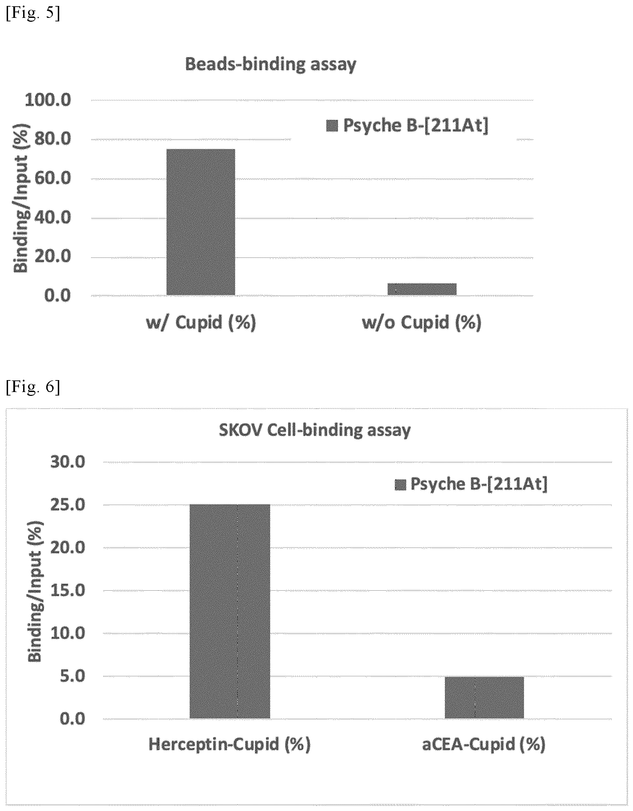

That is, 4 µL of Herceptin-V2122 solution or PBS used as a control was dispensed into a 1.5-mL tube. Into the tube, 10 µL (corresponding to 5.8 kBq) of Psyche-At211 was added, and the obtained mixture was then incubated at room temperature for 5 minutes. Subsequently, 10 µL of Ni-NTA magnetic beads equilibrated with PBS were added to the reaction mixture, and the thus obtained mixture was then incubated at room temperature for 5 minutes. Thereafter, the beads were precipitated and immobilized by using a magnet, and a supernatant was then recovered. The supernatant was washed with 100 µL of PBS twice, and the radiation dose was then counted using a gamma counter. The results are shown in the graph of Fig. 5. It was confirmed that Psyche-At211 was precipitated in a Herceptin-V2122 presence-dependent manner.

### Test Example 3: Confirmation of binding conjugate of astatine-labeled Psyche B and Herceptin-Cupid by using ovarian cancer cells SKOV3

Whether or not astatine-labeled Psyche B has binding ability to Herceptin-V2122, which had previously been bound to HER2-positive SKOV3 cells, was confirmed. As a negative control, CEA-V2122 was used. Psyche B-At211 used herein was prepared according to Example 1(4), whereas Herceptin-V2122 and CEA-V2122 were prepared according to the method described in Japanese Patent Application 2018-247363, as described above. The specific assay method is as follows.

Four 1.5-mL tubes were prepared, and the cells were then dispersed into each tube to a cell density of 0.875 x 10⁶ cells/tube. Herceptin-V2122 was added to two tubes to result in N = 2, whereas CEA-V2122 was added to the remaining two tubes. Subsequently, incubation was carried out on ice for 1 hour, and astatine-labeled Psyche B was then added to the resultant to 10 kBq/tube. Thereafter, the obtained mixture was incubated at room temperature for 30 minutes, while shaking. Finally, the cells were precipitated by centrifugation, and a supernatant was then recovered. The supernatant was washed with PBS twice, and the radiation dose was then counted using a gamma counter. The results are shown in the graph of Fig. 6. The measurement results were Herceptin-V2122-dependent, and it was confirmed that Psyche B-At212 bound to the SKOV3 cells via Herceptin-V2122.

SEQ ID NO: 1 
AEAGITGTWSDQLGDTFIVTAGADGALTGTYENAVGGAESRYVLTGRYDSAPATD
GSGTALGWTVAWKNNSKNAHSATTWSGQYVGGADAKINTQWLLTSGTTNANAW
KSTLVGHDTFTKVKPSAASHHHHHH
SEQ ID NO: 2 (sm3E-scFv sequence) 
QVKLEQSGAEVVKPGASVKLSCKASGFNIKDSYMHWLRQGPGQRLEWIGWIDPE
NGDTEYAPKFQGKATFTTDTSANTAYLGLSSLRPEDTAVYYCNEGTPTGPYYFDY
WGQGTLVTVSSGGGGSGGGGSGGGGSENVLTQSPSSMSVSVGDRVNIACSASSSV
PYMHWLQQKPGKSPKLLIYLTSNLASGVPSRFSGSGSGTDYSLTISSVQPEDAATY
YCQQRSSYPLTFGGGTKLEIK
SEQ ID NO: 3 
QVKLEQSGAEVVKPGASVKLSCKASGFNIKDSYMHWLRQGPGQRLEWIGWIDPE
NGDTEYAPKFQGKATFTTDTSANTAYLGLSSLRPEDTAVYYCNEGTPTGPYYFDY
WGQGTLVTVSSGGGGSGGGGSGGGGSENVLTQSPSSMSVSVGDRVNIACSASSSV
PYMHWLQQKPGKSPKLLIYLTSNLASGVPSRFSGSGSGTDYSLTISSVQPEDAATY
YCQQRSSYPLTFGGGTKLEIKGGGGSGGGGAEAGITGTWSDQLGDTFIVTAGADG
ALTGTYENAVGGAESRYVLTGRYDSAPATDGSGTALGWTVAWKNNSKNAHSATT
WSGQYVGGADAKINTQWLLTSGTTNANAWKSTLVGHDTFTKVKPSAASHHHHH
H
SEQ ID NO: 4 
MKYLLPTAAAGLLLLAAQPAMAQVKLEQSGAEVVKPGASVKLSCKASGFNIKDS
YMHWLRQGPGQRLEWIGWIDPENGDTEYAPKFQGKATFTTDTSANTAYLGLSSLR
PEDTAVYYCNEGTPTGPYYFDYWGQGTLVTVSSGGGGSGGGGSGGGGSENVLTQ
SPSSMSVSVGDRVNIACSASSSVPYMHWLQQKPGKSPKLLIYLTSNLASGVPSRFS
GSGSGTDYSLTISSVQPEDAATYYCQQRSSYPLTFGGGTKLEIKGGGGSGGGGAEA
GITGTWSDQLGDTFIVTAGADGALTGTYENAVGGAESRYVLTGRYDSAPATDGSG
TALGWTVAWKNNSKNAHSATTWSGQYVGGADAKINTQWLLTSGTTNANAWKST
LVGHDTFTKVKPSAASHHHHHH
SEQ ID NO: 5 
ATGAAATATCTGCTGCCGACCGCAGCAGCGGGTCTGCTGCTGCTGGCAGCACA
GCCTGCAATGGCACAGGTTAAACTGGAACAGAGCGGTGCCGAAGTTGTTAAAC
CGGGTGCAAGCGTTAAACTGAGCTGTAAAGCAAGCGGCTTTAACATCAAAGAT
AGCTATATGCATTGGCTGCGTCAGGGTCCGGGTCAGCGTCTGGAATGGATTGGT
TGGATTGATCCGGAAAATGGTGATACCGAATATGCACCGAAATTTCAGGGTAAA
GCAACCTTTACCACCGATACCAGCGCAAATACCGCATATCTGGGTCTGAGCAGC
CTGCGTCCGGAAGATACCGCAGTGTATTATTGTAATGAAGGCACCCCGACCGGT
CCGTATTATTTCGATTATTGGGGTCAGGGCACCCTGGTTACCGTTAGCAGCGGTG
GTGGTGGTAGTGGTGGCGGTGGTTCAGGCGGTGGCGGTAGCGAAAATGTTCTG
ACCCAGAGCCCGAGCAGCATGAGCGTTAGCGTTGGTGATCGTGTTAATATTGCA
TGTAGCGCAAGCAGCAGCGTTCCGTACATGCACTGGCTGCAGCAGAAACCGGG
TAAAAGCCCGAAACTGCTGATTTATCTGACCAGCAATCTGGCAAGCGGTGTTCC
GAGCCGTTTTAGCGGTAGCGGTAGTGGCACCGATTATAGCCTGACCATTAGCAG
CGTGCAGCCTGAAGATGCAGCAACCTATTATTGTCAGCAGCGTAGCAGTTATCC
GCTGACCTTTGGTGGTGGCACCAAACTGGAAATTAAAGGGGGTGGTGGCTCAG
GTGGCGGAGGTGCAGAAGCAGGTATTACCGGTACATGGTCAGATCAGCTGGGT
GATACCTTTATTGTTACCGCAGGCGCAGATGGTGCACTGACCGGCACCTATGAA
AATGCAGTTGGTGGTGCAGAAAGCCGTTATGTGCTGACCGGTCGTTATGATAGC
GCACCGGCAACCGATGGTAGCGGCACCGCACTGGGTTGGACCGTTGCATGGAA
 
AAATAACAGCAAAAATGCACATAGCGCAACCACCTGGTCAGGTCAGTATGTGG
GTGGTGCCGATGCCAAAATTAACACCCAGTGGCTGCTGACCAGCGGTACAACC
AATGCAAATGCCTGGAAAAGTACCCTGGTTGGTCATGATACATTCACCAAAGTT
AAACCGAGCGCAGCAAGCCATCATCATCACCATCATTAA
SEQ ID NO: 6 
MKAGSDRTKLEKDVMAQRQTFAQKAQAFEQDRARRSNEERGKLVTRIQTAVKSV
ANSQDIDLVVDANAVAYNSSDVKDITADVLKQVK
SEQ ID NO: 7 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDY
WGQGTLVTVSSGGGGSGGGGSGGGGSGGGGSDIQMTQSPSSLSASVGDRVTITCR
ASQDVNTAVAWYQQKPGKAPKLLIYSASFLYSGVPSRFSGSRSGTDFTLTISSLQPE
DFATYYCQQHYTTPPTFGQGTKVEIK
SEQ ID NO: 8 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDY
WGQGTLVTVSSGGGGSGGGGSGGGGSGGGGSDIQMTQSPSSLSASVGDRVTITCR
ASQDVNTAVAWYQQKPGKAPKLLIYSASFLYSGVPSRFSGSRSGTDFTLTISSLQPE
DFATYYCQQHYTTPPTFGQGTKVEIKGGGGGSGGGGGAEAGITGTWSDQLGDTFI
VTAGADGALTGTYENAVGGAESRYVLTGRYDSAPATDGSGTALGWTVAWKNNSK
NAHSATTWSGQYVGGADAKINTQWLLTSGTTNANAWKSTLVGHDTFTKVKPSAA
SHHHHHH
SEQ ID NO: 9 MKYLLPTAAAGLLLLAAQPAMAEVQLVESGGGLVQPGGSLRLSCAASGFNIKDTY 
IHWVRQAPGKGLEWVARIYPTNGYTRYADSVKGRFTISADTSKNTAYLQMNSLRA
EDTAVYYCSRWGGDGFYAMDYWGQGTLVTVSSGGGGSGGGGSGGGGSGGGGSD
IQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKGGGGGSG
GGGGAEAGITGTWSDQLGDTFIVTAGADGALTGTYENAVGGAESRYVLTGRYDS
APATDGSGTALGWTVAWKNNSKNAHSATTWSGQYVGGADAKINTQWLLTSGTT
NANAWKSTLVGHDTFTKVKPSAASHHHHHH
SEQ ID NO: 10

ATGAAATATCTGCTGCCGACCGCAGCAGCGGGTCTGCTGCTGCTGGCAGCACA
GCCTGCAATGGCAGAAGTTCAGCTGGTTGAAAGCGGTGGTGGTCTGGTTCAGC
CTGGTGGTAGCCTGCGTCTGAGCTGTGCAGCAAGCGGTTTTAACATTAAAGATA
CCTATATTCATTGGGTGCGTCAGGCACCTGGTAAAGGTCTGGAATGGGTTGCAC
GTATTTATCCGACCAATGGTTATACCCGTTATGCCGATAGCGTTAAAGGTCGTTTT
ACCATTAGCGCAGATACCAGCAAAAATACCGCATACCTGCAGATGAATAGTCTG
CGTGCAGAGGATACCGCAGTGTATTATTGTAGCCGTTGGGGTGGTGATGGTTTT
TATGCAATGGATTATTGGGGTCAGGGCACCCTGGTTACCGTTAGCTCAGGTGGA
GGCGGTTCCGGTGGCGGAGGTTCCGGTGGAGGTGGCTCCGGTGGCGGAGGTTC
CGATATTCAGATGACCCAGAGTCCGAGCAGCCTGAGCGCAAGCGTTGGTGATC
GTGTGACCATTACCTGTCGTGCAAGCCAGGATGTTAATACAGCAGTTGCATGGT
ATCAGCAGAAACCGGGTAAAGCACCGAAACTGCTGATTTATAGCGCAAGCTTT
CTGTATAGCGGTGTTCCGAGCCGTTTTAGCGGTAGCCGTAGCGGCACCGATTTT
ACCCTGACCATTAGCAGCCTGCAGCCGGAAGATTTTGCAACCTATTATTGTCAG
CAGCATTACACCACACCGCCTACCTTTGGCCAGGGCACCAAAGTTGAAATTAA
AGGAGGTGGCGGTGGATCCGGCGGAGGTGGCGGAGCAGAAGCAGGTATTACC
GGTACATGGTCAGATCAGCTGGGTGATACCTTTATTGTTACCGCAGGCGCAGAT
GGTGCACTGACCGGCACCTATGAAAATGCAGTTGGTGGTGCAGAAAGCCGTTA
TGTGCTGACCGGTCGTTATGATAGCGCACCGGCAACCGATGGTAGCGGCACCGC
ACTGGGTTGGACCGTTGCATGGAAAAATAACAGCAAAAATGCACATAGCGCAA

CCACCTGGTCAGGTCAGTATGTGGGTGGTGCCGATGCCAAAATTAACACCCAGT
GGCTGCTGACCAGCGGTACAACCAATGCAAATGCCTGGAAAAGTACCCTGGTT
GGTCATGATACATTCACCAAAGTTAAACCGAGCGCAGCAAGCCATCATCATCAC
CATCATTAA

## Claims

1. A compound represented by the following formula (1) or a salt thereof: 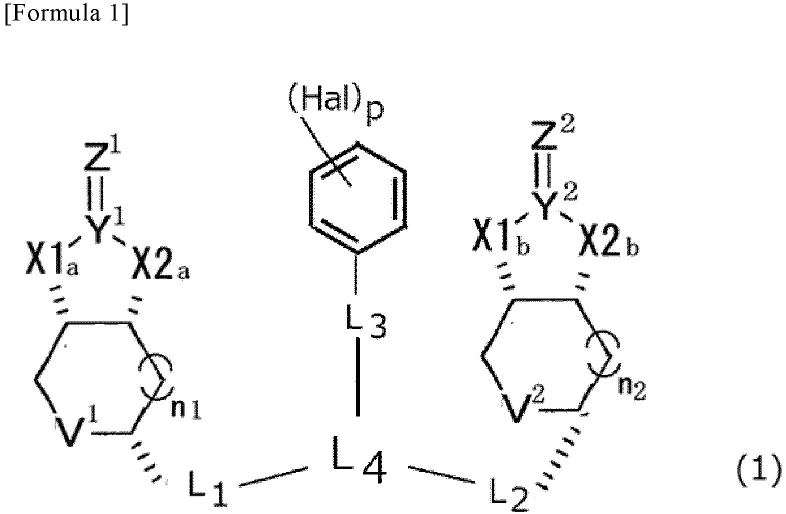 wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻,
n1 and n2 each independently represent an integer of 0 or 1,
L₁, L₂, and L₃ each independently represent a divalent linking group,
L₄ represents a trivalent linking group,
Hal represents a halogen, and
p represents an integer of 1 to 5.

2. The compound according to claim 1, wherein the compound represented by the formula (1) is a compound represented by the following formula (1a) or the following formula (1b): 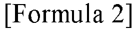 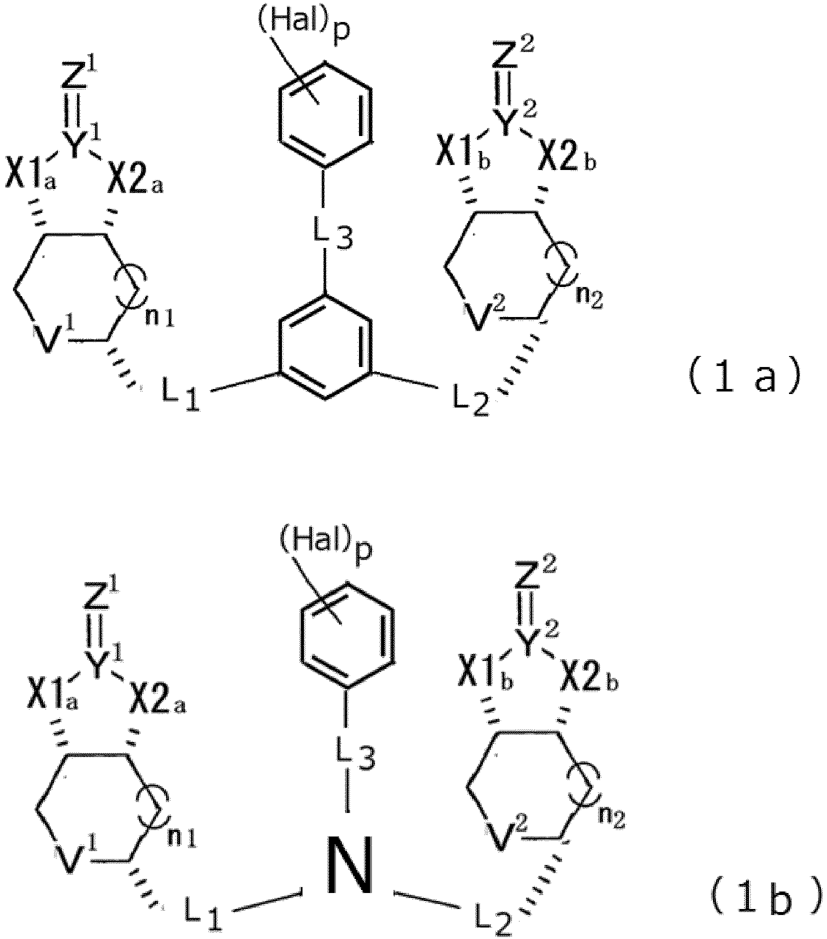 wherein each symbol is as defined in claim 1.

3. The compound or a salt thereof according to claim 1 or 2, which is represented by the following formula (2) or the following formula (3), wherein n1 and n2 are 0. 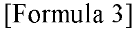 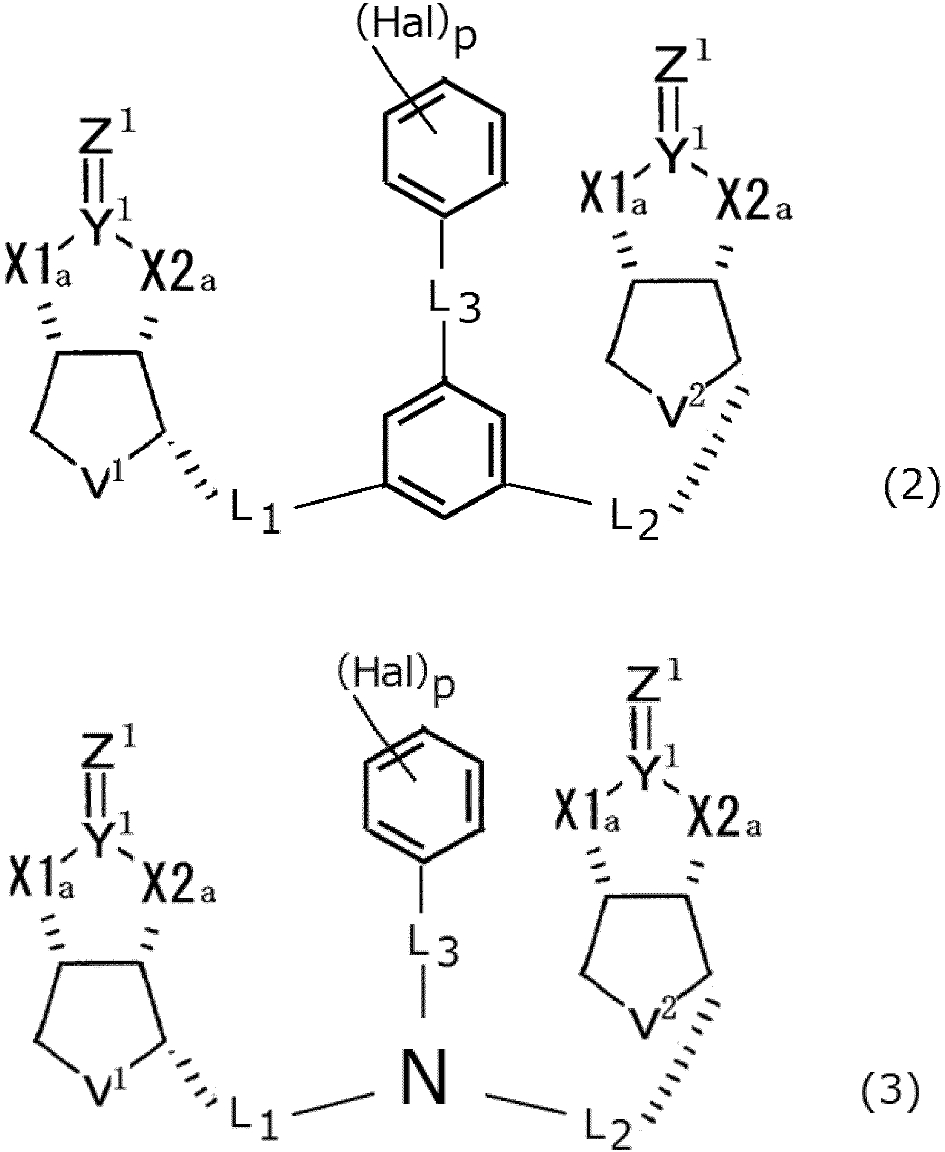 wherein each symbol is as defined in claim 1.

4. The compound or a salt thereof according to any one of claims 1 to 3, wherein X1a, X1b, X2a and X2b represent NH, Y¹ and Y² represent C, Z¹ and Z² represent NH, and V¹ and V² represent S.

5. The compound or a salt thereof according to any one of the above [1] to [4], wherein L₁, L₂, and L₃ each independently represent a divalent linking group consisting of a combination of groups selected from -CONH-, -NHCO-, -COO-, -OCO-, - CO-, -O-, and an alkylene group containing 1 to 10 carbon atoms.

6. The compound or a salt thereof according to any one of claims 1 to 5, wherein
L₁ represents -(CH₂)ml-L₁₁-(CH₂)m₂-L₁₂-*,
L₂ represents *-L₁₃-(CH₂)m₃-L₁₄-(CH₂)m₄-, and
L3 represents L₁₅-L₂₁-L₁₆-,
wherein L₁₁, L₁₂, L₁₃, L₁₄, L₁₅, and L₁₆ each independently represent -CONH-, -NHCO-, - COO-, -OCO-, -CO-, or -O-,
m₁, m₂, m₃, and m₄ each independently represent an integer from 1 to 10,
* represents a binding site with a benzene ring,
L21 represents -CH₂-(OCH₂)ₘ₅-, and
ms represents an integer from 1 to 10.

7. The compound or a salt thereof according to any one of claims 1 to 6, wherein Hal represents I, ¹²⁵I, or ²¹¹At.

8. The compound or a salt thereof according to any one of claims 1 to 7, wherein p is 1.

9. A compound represented by any one of the following formulae:

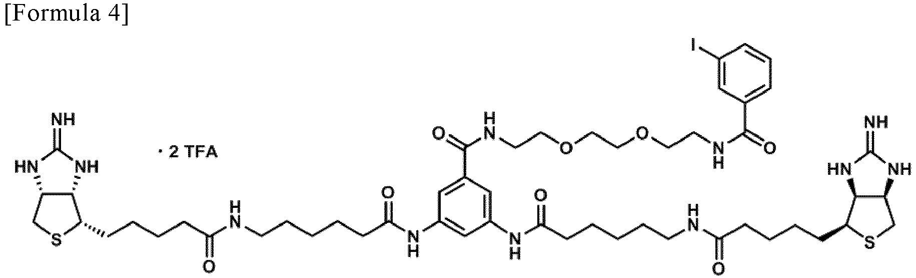

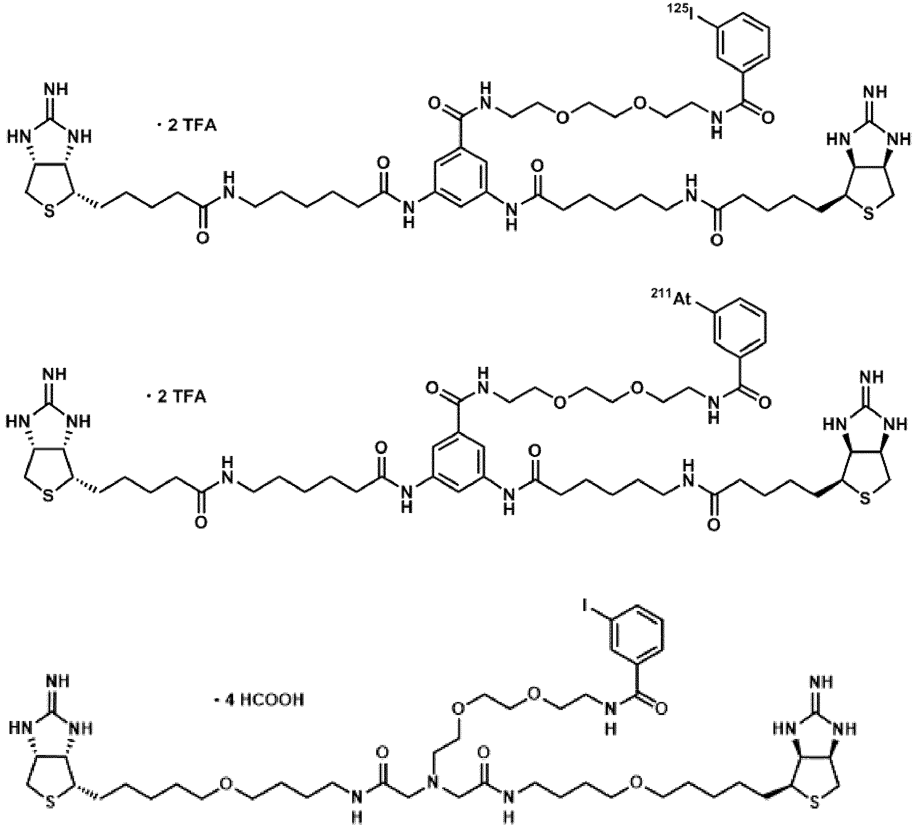

10. A kit for treatment or diagnosis, comprising: (1) the compound according to any one of claims 1 to 9; and (b) a mutant streptavidin-molecular probe conjugate obtained by binding a molecular probe to a mutant streptavidin comprising the amino acid sequence as set forth in SEQ ID NO: 19.

11. A method for producing the compound or a salt thereof according to any one of claims 1 to 9, comprising reacting a compound represented by the following formula (A) with sodium halide or halogen: 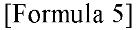 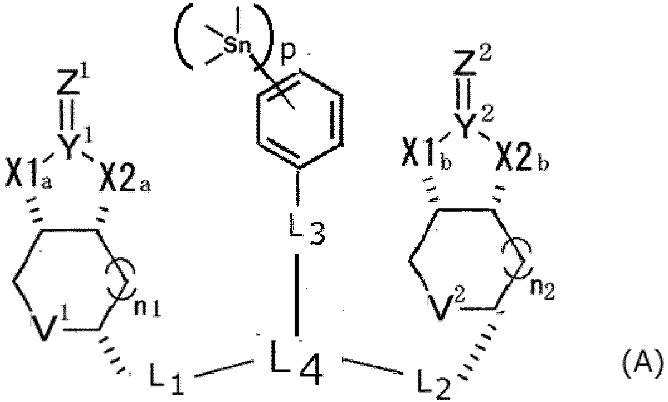 wherein each symbol is as defined in claim 1.

12. A method for producing the compound or a salt thereof according to any one of claims 1 to 9, comprising reacting a compound represented by the following formula (B) with sodium halide or halogen: 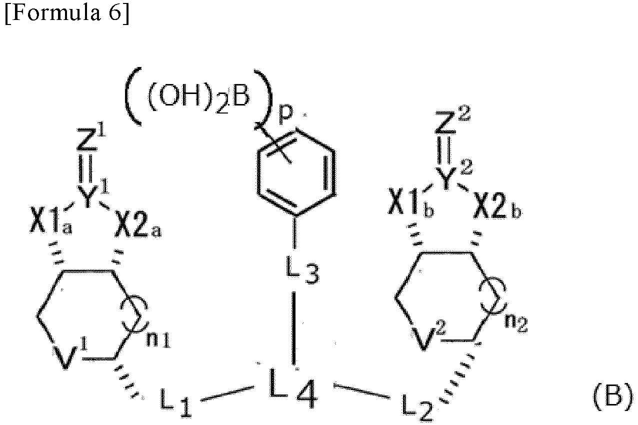 wherein each symbol is as defined in claim 1.
